# EUROPÄISCHE PATENTSCHRIFT

(11) **EP 3 875 056 B1**
(45) Veröffentlichungstag und Bekanntmachung des Hinweises auf die Patenterteilung: **04.05.2022**
(21) Anmeldenummer: 20160298.4
(22) Anmeldetag: 02.03.2020
(51) Int. Cl.: A61C 19/02, A61C 19/00

(54) **VERFAHREN UND SYSTEM ZUR AUFBEREITUNG UND/ODER PFLEGE EINES MEDIZINISCHEN ODER DENTALEN INSTRUMENTS**
METHOD AND SYSTEM FOR THE TREATMENT AND / OR CARE OF A MEDICAL OR DENTAL INSTRUMENT
PROCÉDÉ ET SYSTÈME DE PRÉPARATION ET/OU DE SOIN D'UN INSTRUMENT MÉDICAL OU DENTAIRE

(43) Veröffentlichungstag der Anmeldung: 08.09.2021
(73) Patentinhaber: W & H Dentalwerk Bürmoos GmbH, 5111 Bürmoos (AT)
(72) Erfinder: GALLUSEDER, Florian, 5121 Tarsdorf (AT); REITER, Michael, 5061 Elsbethen (AT)
(74) Vertreter: Benda, Ralf

(56) Entgegenhaltungen:
- EP-A1- 1 652 488
- EP-A1- 1 749 502
- EP-A1- 2 138 127
- EP-A1- 2 514 386

## Beschreibung

Die vorliegende Erfindung betrifft ein Verfahren und ein System zur Aufbereitung und/ oder Pflege eines medizinischen oder dentalen Instruments.

Eine Behandlung eines medizinischen oder dentalen Instruments nach dessen Verwendung umfasst gemäß gegenwärtigen Anforderungen mehrere Schritte, zum Beispiel eine Reinigung und/ oder Desinfektion, eine Pflege und eine Sterilisation. Häufig werden diese Behandlungsschritte von verschiedenen Aufbereitungs- und/ oder Pflegegeräten durchgeführt, wobei ein Anwender das medizinische oder dentale Instrument nach jedem Behandlungsschritt aus dem entsprechenden Gerät entnehmen und/oder davon trennen und in ein folgendes Gerät einbringen und/ oder damit verbinden muss.

Da die verschiedenen Aufbereitungs- und/ oder Pflegegeräte und verschiedene medizinische oder dentale Instrumente oftmals unterschiedliche Schnittstellen haben, ist es zur Herstellung einer Verbindung zwischen einem Aufbereitungs- und/ oder Pflegegerät und einem medizinischen oder dentalen Instrument notwendig, einen Verbindungsadapter vorzusehen. Derartige Verbindungsadapter sind insbesondere dann notwendig, wenn ein Aufbereitungs- und/ oder Pflegemittel in das Innere eines medizinischen oder dentalen Instruments geleitet werden soll, um das Innere oder Bauteile im Inneren des medizinischen oder dentalen Instruments aufzubereiten und/ oder zu pflegen. Beim Wechsel von einem Aufbereitungs- und/ oder Pflegegeräte zu einem anderen Aufbereitungs- und/ oder Pflegegeräte muss ein Anwender somit zusätzlich auch jeweils die Verbindungsadapter austauschen, so dass das medizinische oder dentale Instrument mit dem folgenden Aufbereitungs- und/ oder Pflegegerät verbunden werden kann.

Aus den Patentanmeldungen EP 2 138 127 A1 und EP 1 652 488 A1 sind jeweils Aufbereitungs- und/ oder Pflegevorrichtungen bekannt, bei denen die zu reinigenden und/ oder pflegenden, medizinischen oder dentalen Instrumente mittels Verbindungsadaptern lösbar mit den Aufbereitungs- und/ oder Pflegevorrichtungen verbindbar sind.

Die Patentanmeldung EP 2 514 386 A1 offenbart einen Verbindungsadapter, der ein zu reinigendes und/ oder pflegendes, medizinisches oder dentales Instrument lösbar mit einer Aufbereitungs- und/ oder Pflegevorrichtung und mit einer Versorgungseinheit verbinden kann. Aus der Patentanmeldung EP 1 749 502 A1 sind ein Pflege- oder Reinigungsgerät für medizinische Instrumente, ein Verbindungsadapter sowie ein Verfahren zum Betrieb eines Pflege- oder Reinigungsgeräts bekannt.

Der vorliegenden Erfindung liegt daher die Aufgabe zugrunde, einem Anwender den Ablauf eines Verfahrens zur Aufbereitung und/ oder Pflege eines medizinischen oder dentalen Instruments zu erleichtern und den Aufbereitungs- und/ oder Pflegeprozess in seiner Gesamtheit zeitlich zu verkürzen.

Diese Aufgabe wird gemäß der vorliegenden Erfindung durch ein Verfahren zur Aufbereitung und/ oder Pflege eines medizinischen oder dentalen Instruments mit den Merkmalen des Anspruchs 1 und ein System zur Aufbereitung und/ oder Pflege eines medizinischen oder dentalen Instruments mit den Merkmalen des Anspruchs 8 gelöst. Besonders vorteilhafte Ausführungsformen sind in den Unteransprüchen angeführt.

Ein Vorteil des Verfahrens zur Aufbereitung und/ oder Pflege eines medizinischen oder dentalen Instruments und des Systems zur Aufbereitung und/ oder Pflege eines medizinischen oder dentalen Instruments besteht darin, dass der Verbindungsadapter mit mehreren Aufbereitungs- und/ oder Pflegevorrichtungen (operativ) verbindbar ist, so dass der Verbindungsadapter und das daran befestigte medizinische oder dentale Instrument während des Verfahrens zur Aufbereitung und/ oder Pflege beim Wechsel von einer Aufbereitungs- und/ oder Pflegevorrichtung zu einer anderen Aufbereitungs- und/ oder Pflegevorrichtung nicht getrennt werden müssen und somit insbesondere als (lösbare) Einheit mit zumindest zwei während eines Verfahrens verwendeter Aufbereitungs- und/ oder Pflegevorrichtungen verbindbar sind. Vorzugsweise ist der Verbindungsadapter oder die Verbindungsadapter-Instrumenten-Einheit mit allen Aufbereitungs- und/ oder Pflegevorrichtung des Verfahrens verbindbar, so dass während des gesamten Aufbereitungs- und/ oder Pflegeverfahrens keine Trennung der Verbindungsadapter-Instrumenten-Einheit notwendig ist. Dies vereinfacht für den Anwender den Ablauf des Verfahrens zur Aufbereitung und/ oder Pflege und hilft Zeit zu sparen. Auch ist in vorteilhafter Weise die Anzahl der benötigten Verbindungsadapter reduziert.

Das Verbinden des medizinischen oder dentalen Instruments mit dem Verbindungsadapter und/ oder das Verbinden des Verbindungsadapters mit der ersten oder zweiten Aufbereitungs- und/ oder Pflegevorrichtung, so wie es zum Beispiel in den Schritten b) und e) des vorstehenden Verfahrens beschrieben ist, wird insbesondere jeweils durch das Zusammenfügen von Steckverbindungen bewirkt. Die Steckverbindungen sind insbesondere durch Kupplungselemente oder Steckelemente an dem Verbindungsadapter, dem Instrument und der ersten und zweiten Aufbereitungs- und/ oder Pflegevorrichtung gebildet, die im Folgenden im Detail beschrieben sind. Auch das Trennen des medizinischen oder dentalen Instruments von dem Verbindungsadapter und/ oder das Lösen des Verbindungsadapters von der ersten oder zweiten Aufbereitungs- und/ oder Pflegevorrichtung, so wie es zum Beispiel in dem Schritt d) des Verfahrens beschrieben ist, ist durch die Steckverbindungen bewirkt.

Das Trennen des Verbindungsadapters gemeinsam mit dem daran befestigten medizinischen oder dentalen Instrument von der ersten Aufbereitungs- und/ oder Pflegevorrichtung, so wie es zum Beispiel im Schritt d) des vorstehenden Verfahrens beschrieben ist, bedeutet insbesondere, dass der Verbindungsadapter und das Instrument eine Einheit bilden, die von der ersten Aufbereitungs- und/ oder Pflegevorrichtung gelöst wird. Insbesondere werden der Verbindungsadapter und das medizinische oder dentale Instrument dabei nicht voneinander gelöst. Insbesondere bleiben der Verbindungsadapter und das medizinische oder dentale Instrument bei der Trennung von der ersten Aufbereitungs- und/ oder Pflegevorrichtung so miteinander verbunden, wie sie bei der vorher stattfindenden Aufbereitung und/ oder Pflege durch die erste Aufbereitungs- und/ oder Pflegevorrichtung, die zum Beispiel im Schritt c) des vorstehenden Verfahrens beschrieben ist, verbunden sind. Der Verbindungsadapter und das Instrument werden somit während des Verfahrens in vorteilhafter Weise gemeinsam, als (ungetrennte) Einheit gehandhabt.

Das Verbinden des Verbindungsadapters gemeinsam mit dem daran befestigten medizinischen oder dentalen Instrument mit oder an der zweiten Aufbereitungs- und/ oder Pflegevorrichtung, so wie es zum Beispiel im Schritt e) des vorstehenden Verfahrens beschrieben ist, bedeutet insbesondere, dass der Verbindungsadapter und das Instrument eine Einheit bilden, die an der zweiten Aufbereitungs- und/ oder Pflegevorrichtung befestigt wird. Insbesondere werden der Verbindungsadapter und das medizinische oder dentale Instrument dabei nicht voneinander gelöst. Insbesondere bleiben der Verbindungsadapter und das medizinische oder dentale Instrument bei der Verbindung mit der zweiten Aufbereitungs- und/ oder Pflegevorrichtung so miteinander verbunden, wie sie bei der vorher stattfindenden Aufbereitung und/ oder Pflege durch die erste Aufbereitungs- und/ oder Pflegevorrichtung, die zum Beispiel im Schritt c) des vorstehenden Verfahrens beschrieben ist, und/ oder bei der Trennung von der ersten Aufbereitungs- und/ oder Pflegevorrichtung, so wie zum Beispiel im Schritt d) des vorstehenden Verfahrens, verbunden sind. Der Verbindungsadapter und das Instrument werden somit während des Verfahrens in vorteilhafter Weise gemeinsam, als (ungetrennte) Einheit gehandhabt.

Vorzugsweise umfasst das Verbinden des medizinischen oder dentalen Instruments über den Verbindungsadapter mit der ersten Aufbereitungs- und/ oder Pflegevorrichtung, so wie es zum Beispiel im Schritt b) des vorstehenden Verfahrens beschrieben ist, folgende Teilschritte:
b1) (lösbares) Verbinden des medizinischen oder dentalen Instruments, insbesondere des Kupplungsendes des Instruments, mit dem Verbindungsadapter;
b2) (lösbares) Verbinden des Verbindungsadapters gemeinsam mit dem daran befestigten medizinischen oder dentalen Instrument mit der ersten Aufbereitungs- und/ oder Pflegevorrichtung, insbesondere mit einem Kupplungsstück der ersten Aufbereitungs- und/ oder Pflegevorrichtung.

Vorzugsweise werden zuerst der Schritt b1) und anschließend der Schritt b2) durchgeführt, wodurch die Handhabung für den Anwender besonders einfach ist. Alternativ ist es auch möglich, zuerst den Verbindungsadapter (lösbar) an der ersten Aufbereitungs- und/ oder Pflegevorrichtung zu befestigen, insbesondere an einem Kupplungsstück der ersten Aufbereitungs- und/ oder Pflegevorrichtung, und darauffolgend das medizinische oder dentale Instrument, insbesondere dessen Kupplungsende, (lösbar) mit dem Verbindungsadapter zu verbinden.

Durch das Verbinden des Verbindungsadapters und des Instruments, so wie beispielsweise in Schritt b1) beschrieben, bilden der Verbindungsadapter und das Instrument somit eine Einheit, die in mehreren, unterschiedlichen Aufbereitungs- und/ oder Pflegevorrichtungen oder -schritten eines Verfahrens zur Aufbereitung und/ oder Pflege verwendet wird ohne dabei (zwischenzeitlich) getrennt zu werden. Der Verbindungsadapter und das Instrument werden somit während des (gesamten) Verfahrens in vorteilhafter Weise gemeinsam, als (ungetrennte) Einheit gehandhabt, insbesondere beim Verbinden mit der ersten Aufbereitungs- und/ oder Pflegevorrichtung, beispielsweise in Schritt b2) des vorstehenden Verfahrens, und besonders bevorzugt auch bei der Aufbereitung und/ oder Pflege, beispielsweise in den Schritten c) und f) des vorstehenden Verfahrens, beim Trennen des Verbindungsadapters mit dem Instrument von der ersten Aufbereitungs- und/ oder Pflegevorrichtung, beispielsweise im Schritt d) des vorstehenden Verfahrens, und beim Verbinden des Verbindungsadapters mit dem Instrument an der zweiten Aufbereitungs- und/ oder Pflegevorrichtung, beispielsweise im Schritt e) des vorstehenden Verfahrens. Insbesondere bleiben der Verbindungsadapter und das medizinische oder dentale Instrument bei einem oder mehreren nachfolgenden Schritten des Verfahrens zur Aufbereitung- und/ oder Pflege und/ oder beim Wechsel zwischen unterschiedlichen Aufbereitungs- und/ oder Pflegevorrichtungen so miteinander verbunden wie sie in einem oder mehreren vorherigen Schritten des Verfahrens zur Aufbereitung- und/ oder Pflege oder in einem bereits genutzten Aufbereitungs- und/ oder Pflegevorrichtung miteinander verbunden waren.

Vorzugsweise wird am oder nach dem Ende des Verfahrens zur Aufbereitung und/ oder Pflege, insbesondere nach Abschluss des letzten Aufbereitungs- und/ oder Pflegeschrittes des medizinischen oder dentalen Instruments, beispielsweise nach Schritt f) des vorstehenden Verfahrens, das medizinische oder dentale Instrument von dem Verbindungsadapter getrennt.

Bevorzugt wird zuerst der Verbindungsadapter gemeinsam mit dem daran befestigten medizinischen oder dentalen Instrument von der zuletzt verwendeten, gemäß dem vorstehenden Verfahren zum Beispiel zweiten Aufbereitungs- und/ oder Pflegevorrichtung getrennt. Anschließend wird das medizinische oder dentale Instrument von dem Verbindungsadapter gelöst, insbesondere die im Vorstehenden beschriebene Einheit des Verbindungsadapters und des Instruments getrennt. Diese genannte Reihenfolge ist für einen Anwender besonders einfach zu handhaben.

Alternativ ist es auch möglich, zuerst das medizinische oder dentale Instrument von dem Verbindungsadapter zu trennen, so dass der Verbindungsadapter noch an dem zuletzt verwendeten, zum Beispiel zweiten Aufbereitungs- und/ oder Pflegevorrichtung verbleibt. Der Verbindungsadapter wird anschließend (ohne das Instrument) von der zweiten Aufbereitungs- und/ oder Pflegevorrichtung getrennt.

Vorzugsweise umfasst das medizinische oder dentale Instrument eine hohle Außenhülse, in welcher zumindest ein Bauteil angeordnet ist, wobei die Aufbereitung und/ oder Pflege des medizinischen oder dentalen Instruments durch die erste Aufbereitungs- und/ oder Pflegevorrichtung und/ oder durch die zweite Aufbereitungs- und/ oder Pflegevorrichtung eine Aufbereitung und/ oder Pflege des zumindest einen im Inneren der hohlen Außenhülse angeordneten Bauteils umfasst. Insbesondere umfasst die Aufbereitung und/ oder Pflege des Inneren der Außenhülse die Leitung eines Aufbereitungs- und/ oder Pflegemediums in Richtung oder zu dem zumindest einen Bauteil. Das zumindest eine Bauteil im Inneren der Außenhülse umfasst zum Beispiel zumindest eines der folgenden Bauteile: eine Luftleitung; eine Wasserleitung; eine Medienleitung; eine Fluidleitung; ein Zahnrad; ein Getriebe; ein Lager für ein in Bewegung versetzbares Bauteil des medizinischen oder dentalen Instruments; eine Werkzeughalterung; eine Werkzeuglösevorrichtung; und ähnliche Bauteile.

Das medizinische oder dentale Instrument wird durch den Verbindungsadapter insbesondere mit einem Kupplungsstück der ersten oder zweiten Aufbereitungs- und/ oder Pflegevorrichtung verbunden. Das Kupplungsstück ist insbesondere als Steckelement oder als Teil der Steckverbindung zur Verbindung mit dem Verbindungsadapter, so wie sie im Vorstehenden bereits beschrieben wurden, ausgebildet. Das Kupplungsstück umfasst vorzugsweise zumindest eine Medienleitung zur Leitung eines Aufbereitungs- und/ oder Pflegemediums, wobei die Medienleitung insbesondere in einer Öffnung an einer Oberfläche des Kupplungsstücks, zum Beispiel an einer Oberfläche einer Kupplungsaufnahme des Kupplungsstücks endet.

Vorzugsweise ist das Kupplungsstück der ersten und/ oder zweiten Aufbereitungs- und/ oder Pflegevorrichtung derart ausgebildet, dass es ein Aufbereitungs- und/ oder Pflegemedium in das Innere der hohlen Außenhülse des aufzubereitenden und/ oder zu pflegenden medizinischen oder dentalen Instruments leitet, insbesondere in oder in Richtung zumindest eines Bauteils in der hohlen Außenhülse, so wie dies im Vorstehenden bereits beschrieben ist. Damit ist es in vorteilhafter Weise möglich, das Innere des Instruments zu reinigen oder aufzubereiten. Bevorzugt ist das Kupplungsstück dazu derart geformt, dass es in der hohlen Außenhülse des medizinische oder dentale Instrument aufnehmbar ist.

Das Kupplungsstück umfasst zum Beispiel eine Bohrung, die derart angeordnet ist, dass, wenn das Kupplungsstück mit dem Instrument verbunden ist, ein Aufbereitungs- und/ oder Pflegemedium in das Innere der hohlen Außenhülse, insbesondere in oder in Richtung des zumindest eines Bauteils, fließt. Besonders bevorzugt ist die Bohrung derart an dem Kupplungsstück angeordnet, dass die Bohrung, wenn das Kupplungsstück mit dem Instrument verbunden ist, in der hohlen Außenhülse aufgenommen ist. Alternativ oder zusätzlich umfasst Kupplungsstück zum Beispiel eine Bohrung, die, wenn das Instrument an dem Kupplungsstück befestigt ist, mit einer Medienleitung des medizinischen oder dentalen Instruments verbunden ist, um ein Aufbereitungs- und/ oder Pflegemedium in die Medienleitung zu leiten.

Die zumindest eine Medienleitung des Kupplungsstücks ist vorzugsweise mit einer Medienquelle verbunden, die das Aufbereitungs- und/ oder Pflegemedium zur Verfügung stelle und/ oder aufbewahrt. Die zumindest eine Medienleitung des Kupplungsstücks erstreckt sich vorzugsweise von dem Kupplungsstück durch die erste oder zweite Aufbereitungs- und/ oder Pflegevorrichtung bis zu der Medienquelle oder die zumindest eine Medienleitung des Kupplungsstücks ist über eine Zufuhrleitung mit der Medienquelle verbunden. Die Medienquelle kann zum Beispiel einen Behälter für das Aufbereitungs- und/ oder Pflegemedium und/ oder einen Anschluss an eine Medienversorgung, insbesondere eine Wasserversorgung, einen Verdampfer oder einen Kompressor umfassen.

Damit der Verbindungsadapter jeweils mit dem Kupplungsstück der ersten und der zweiten Aufbereitungs- und/ oder Pflegevorrichtung verbindbar ist, sodass insbesondere ein im Vorstehenden beschriebenes Verfahren zur Aufbereitung und/ oder Pflege durchführbar ist, sind die Kupplungsstücke der ersten und zweiten Aufbereitungs- und/ oder Pflegevorrichtung ident oder im Wesentlichen ident ausgebildet. Alternativ unterscheiden sich die Kupplungsstücke der erste und zweiten Aufbereitungs- und/ oder Pflegevorrichtung (geringfügig) voneinander, wobei trotz der Unterschiede der Verbindungsadapter an beide Kupplungsstücke funktionsgemäß, insbesondere Fluid übertragend, anschließbar ist, so dass insbesondere ein Aufbereitungs- und/ oder Pflegemedium durch die zumindest eine Medienleitung des Verbindungsadapters von der ersten Aufbereitungs- und/ oder Pflegevorrichtung an das medizinische oder dentale Instrument, insbesondere in dessen Inneres, und von der zweiten Aufbereitungs- und/ oder Pflegevorrichtung an das medizinische oder dentale Instrument, insbesondere in dessen Inneres, geleitet werden kann. Zum Beispiel unterscheiden sich die Kupplungsstücke der ersten und der zweiten Aufbereitungs- und/ oder Pflegevorrichtung in Bezug auf eine oder mehrere der genannten Eigenschaften: Abmessung; Form; Design; Anschlussbild; Material; und ähnlichen Eigenschaften.

Das Aufbereitungs- und/ oder Pflegemedium kann zum Beispiel zumindest eines der folgenden Medien oder Kombinationen davon umfassen: ein Aufbereitungs- oder Reinigungsmedium, insbesondere eine Reinigungsflüssigkeit oder ein Spülmittel, zum Beispiel ein Detergens, ein Tensid oder ein Druckgas; ein Desinfektionsmedium, insbesondere eine Desinfektionsflüssigkeit oder ein Desinfektionsgas, zum Beispiel ein oxidierendes Mittel, ein organisches oder ein Metallionen enthaltendes Mittel; ein Sterilisationsmedium, insbesondere eine gasförmiges Medium wie zum Beispiel Wasserdampf; ein Pflegemedium, zum Beispiel ein Schmiermittel, insbesondere auf Basis von pflanzlichen Stoffen und/ oder Mineralöl, oder ein gasförmiges Medium, zum Beispiel zum Durchblasen des Instruments.

Die Aufbereitung und/ oder Pflege eines medizinischen oder dentalen Instruments gemäß dem Verfahren umfasst zumindest zwei der folgenden Behandlungen oder Kombinationen davon: eine Pflege, insbesondere mit einem im Vorstehenden beschriebenen Pflegemedium; eine Aufbereitung oder Reinigung, insbesondere mit einem im Vorstehenden beschriebenen Aufbereitungs- oder Reinigungsmedium; eine Desinfektion, insbesondere mit einem im Vorstehenden beschriebenen Desinfektionsmedium; eine Sterilisation, insbesondere mit einem im Vorstehenden beschriebenen Sterilisationsmedium. Die zumindest zwei Behandlungen werden insbesondere zeitlich versetzt oder aufeinanderfolgend durchgeführt.

Zumindest zwei Behandlungen (oder Behandlungsschritte) des Verfahrens zur Aufbereitung und/ oder Pflege eines medizinischen oder dentalen Instruments werden durch zwei unterschiedliche Aufbereitungs- und/ oder Pflegevorrichtungen durchgeführt, zum Beispiel durch eine erste Aufbereitungs- und/ oder Pflegevorrichtung und eine zweite Aufbereitungs- und/ oder Pflegevorrichtung, wobei es zur Durchführung des Verfahrens und/ oder der zumindest zwei Behandlungen notwendig ist, das aufzubereitende und/ oder zu pflegende medizinische oder dentale Instrument (zeitlich versetzt) mit der ersten Aufbereitungs- und/ oder Pflegevorrichtung und der zweiten Aufbereitungs- und/ oder Pflegevorrichtung zu verbinden. Die zwei unterschiedlichen Aufbereitungs- und/ oder Pflegevorrichtungen sind vorzugsweise zwei voneinander unabhängige und/ oder voneinander getrennte, insbesondere räumlich voneinander getrennte Vorrichtungen. Es ist jedoch auch denkbar, dass die erste und die zweiten Aufbereitungs- und/ oder Pflegevorrichtung ein Kombinationsgerät mit einem gemeinsamen Gehäuse bilden.

Die zwei unterschiedlichen Aufbereitungs- und/ oder Pflegevorrichtungen umfassen vorzugsweise getrennte Medienquellen und/ oder Medienzuführungen von Betriebsmedien und/ oder Aufbereitungs- und/ oder Pflegemedien. Die zwei unterschiedlichen Aufbereitungs- und/ oder Pflegevorrichtungen umfassen insbesondere (räumlich) getrennte Kammern zur Aufnahme des aufzubereitenden und/ oder zu pflegenden medizinischen oder dentalen Instruments, jeweils mit zumindest einem Kupplungsstück zum Anschluss des Instruments, so dass es zur Durchführung des Verfahrens und/ oder der zumindest zwei Behandlungen notwendig ist, das Instrument (zeitlich versetzt) in die Kammer der ersten Aufbereitungs- und/ oder Pflegevorrichtung und der zweiten Aufbereitungs- und/ oder Pflegevorrichtung einzubringen und insbesondere das Instrument mit dem Kupplungsstück der jeweiligen Kammer zu verbinden.

Die unterschiedlichen Aufbereitungs- und/ oder Pflegevorrichtungen umfassen gemäß dem Verfahren zumindest zwei der folgenden Geräte oder Kombinationen davon: ein Pflegegerät, das insbesondere ein Pflegemedium, vorzugsweise wie im Vorstehenden beschrieben, an das medizinische oder dentale Instrument abgibt; ein Aufbereitungs- oder Reinigungsgerät, das insbesondere ein Aufbereitungs- oder Reinigungsmedium, vorzugsweise wie im Vorstehenden beschrieben, an das medizinische oder dentale Instrument abgibt; ein Desinfektionsgerät das insbesondere ein Desinfektionsmedium, vorzugsweise wie im Vorstehenden beschrieben, an das medizinische oder dentale Instrument abgibt; einen Sterilisator oder Autoklaven, der ein Sterilisationsmedium, vorzugsweise wie im Vorstehenden beschrieben, an das medizinische oder dentale Instrument abgibt.

Insbesondere versorgen die erste und zweite Aufbereitungs- und/ oder Pflegevorrichtung gemäß dem Verfahren bzw. während der zumindest zwei unterschiedlichen Behandlungsschritte des Verfahrens das aufzubereitende und/ oder zu pflegende medizinische oder dentale Instrument mit zumindest einem unterschiedlichen Aufbereitungs- und/ oder Pflegemedium. Die erste und zweite Aufbereitungs- und/ oder Pflegevorrichtung unterziehen das Instrument unterschiedlichen Behandlungen, wie sie insbesondere im Vorstehenden beschrieben sind, wodurch in vorteilhafter Weise eine umfassende Aufbereitung und/ oder Pflege des medizinischen oder dentalen Instruments erzielt wird.

Das medizinische oder dentale Instrument umfasst insbesondere Hohlinstrumente, zum Beispiel Instrumente mit einer hohlen Außenhülse, in deren Inneren zumindest ein Bauteil angeordnet ist. Vorzugsweise ist das Innere des medizinischen oder dentalen Instruments und/ oder das zumindest eine Bauteil durch die erste und/ oder zweite Aufbereitungs- und/ oder Pflegevorrichtung aufzubereiten und/ oder zu pflegen. Das medizinische oder dentale Instrument umfasst zum Beispiel ein gerades, gebogenes oder gewinkeltes Handstück oder Winkelstück oder ein endoskopisches Instrument.

Das medizinische oder dentale Instrument umfasst insbesondere ein Kupplungsende, das zur Verbindung mit der ersten und zweiten Aufbereitungs- und/ oder Pflegevorrichtung vorgesehen ist. Das Kupplungsende ist vorzugsweise als Kupplungsaufnahme, zum Beispiel als Kupplungsrohr, zur Aufnahme eines Kupplungsfortsatzes ausgebildet. Alternativ kann das Kupplungsende einen oder mehrere Fortsätze aufweisen, zum Beispiel zumindest einen Abschnitt einer durch das Instrument verlaufenden Medienleitung und/ oder einen elektrischen Kontakt, der/ die sich insbesondere von dem Kupplungsende erstreckt/ erstrecken. Alternativ kann das Kupplungsende des medizinischen oder dentalen Instruments durch eine Drehkupplung gebildet sein, die lösbar mit dem Instrument verbunden ist, derart, dass das Instrument relativ zur Drehkupplung drehbar ist. Das vom Instrument abgewandte Ende der Drehkupplung ist vorzugsweise mit einem oder mehreren Fortsätzen versehen, zum Beispiel mit zumindest einem Abschnitt einer durch das Instrument verlaufenden Medienleitung und/ oder einem elektrischen Kontakt, der/ die sich insbesondere vom Ende der Drehkupplung erstreckt/ erstrecken.

Das Kupplungsende des Instruments, zum Beispiel die Kupplungsaufnahme oder zumindest einer der Fortsätze, weisen zumindest eine Öffnung auf, durch die ein Aufbereitungs- und/ oder Pflegemedium in das Inneres des Instruments, insbesondere in das Innere einer hohlen Außenhülse und/ oder zu einem in der Außenhülse angeordneten Bauteil und/ oder in eine Medienleitung des medizinischen oder dentalen Instruments leitbar ist, wodurch in vorteilhafter Weise auch das Innere des Instruments aufbereitet und/ oder gepflegt werden kann. Das Kupplungsende kann zum Beispiel ein Gewinde, insbesondere ein Außengewinde, zur Verbindung mit dem Verbindungsadapter aufweisen. Die Kupplungsaufnahme kann zum Beispiel als Teil einer Steckverbindung oder als Steckaufnahme zur reibschlüssigen und/ oder formschlüssigen Verbindung mit dem Verbindungsadapter ausgebildet sein, so wie dies auch im Vorstehenden bereits erwähnt ist.

Der Verbindungsadapter umfasst zumindest eine Medienleitung für ein Aufbereitungs- und/ oder Pflegemedium, insbesondere zum Durchleiten eines Aufbereitungs- und/ oder Pflegemediums. Vorzugsweise erstreckt sich die zumindest eine Medienleitung durch den Verbindungsadapter, insbesondere entlang einer Längsachse des Verbindungsadapters, wodurch in vorteilhafter Weise eine zuverlässige Übertragung des Mediums bewirkt ist. Vorzugsweise verbindet die zumindest eine Medienleitung eine erste Öffnung an einer Oberfläche des Verbindungsadapters mit einer zweiten Öffnung an einer Oberfläche des Verbindungsadapters, wobei die erste und die zweite Öffnung voneinander beabstandet sind, insbesondere an entgegengesetzten Ende des Verbindungsadapters angeordnet sind.

Vorzugsweise ist der Verbindungsadapter, insbesondere die zumindest eine Medienleitung dazu vorgesehen, ein Aufbereitungs- und/ oder Pflegemedium von der ersten Aufbereitungs- und/ oder Pflegevorrichtung, insbesondere von deren Kupplungsstück, oder von der zweiten Aufbereitungs- und/ oder Pflegevorrichtung, insbesondere von deren Kupplungsstück, an das medizinische oder dentale Instrument zu leiten. Besonders bevorzugt ist der Verbindungsadapter, insbesondere die zumindest eine Medienleitung dazu vorgesehen, ein Aufbereitungs- und/ oder Pflegemedium in die hohle Außenhülse des mit der ersten oder zweiten Aufbereitungs- und/ oder Pflegevorrichtung verbundenen medizinischen oder dentalen Instruments zu leiten, bevorzugt an zumindest ein Bauteil in der hohlen Außenhülse, wodurch in vorteilhafter Weise auch das Innere des Instruments aufbereitet und/ oder gepflegt werden kann.

Besonders bevorzugt ist der Verbindungsadapter derart ausgebildet, dass durch die Kopplung des Verbindungsadapters mit dem medizinischen oder dentalen Instrument und der ersten oder zweiten Aufbereitungs- und/ oder Pflegevorrichtung eine Fluidverbindung zur Leitung des Aufbereitungs- und/ oder Pflegemediums zwischen der ersten Aufbereitungs- und/ oder Pflegevorrichtung, insbesondere deren Kupplungsstück, und dem aufzubereitenden und/ oder zu pflegenden Instrument herstellbar ist. Besonders bevorzugt bewirkt die Kopplung des Verbindungsadapters mit dem medizinischen oder dentalen Instrument und der ersten oder zweiten Aufbereitungs- und/ oder Pflegevorrichtung eine Fluidverbindung der zumindest einen Medienleitung des Verbindungsadapters mit der zumindest einen Medienleitung der ersten oder zweiten Aufbereitungs- und/ oder Pflegevorrichtung, insbesondere mit deren Kupplungsstücken, und mit dem Instrument, insbesondere mit dem Inneren des Instruments. Besonders bevorzugt sind zur Herstellung der Fluidverbindung die Öffnungen der jeweiligen Medienleitungen des Verbindungsadapters, der ersten oder zweiten Aufbereitungs- und/ oder Pflegevorrichtung und des Instruments miteinander fluidverbunden, wodurch in vorteilhafter Weise eine zuverlässige Medienübertragung erreicht ist.

Vorzugsweise umfasst der Verbindungsadapter einen Körper, der sich entlang einer Längsachse erstreckt. Vorzugsweise ist die zumindest eine Medienleitung in dem Körper angeordnet und/ oder erstreckt sich durch den Körper, wodurch in vorteilhafter Weise eine zuverlässige Medienübertragung gewährleistet ist.

Vorzugsweise umfasst der Verbindungsadapter mehrere, zum Beispiel zwei Medienleitungen, die sich durch den Körper des Verbindungsadapters erstrecken, so dass in vorteilhafter Weise eine Vielzahl von Medien übertragbar sind. Vorzugsweise ist eine der mehreren Medienleitungen zur Leitung eines Pflegemediums und eine andere der zwei Medienleitungen zur Leitung eines Aufbereitungsmediums vorgesehen, wodurch in vorteilhafter Weise eine Vermischung unterschiedlicher Medien vermieden wird. Vorzugsweise ist eine Öffnung einer der mehreren Medienleitungen zentrisch (in Bezug auf die Längsachse des Verbindungsadapters) angeordnet, wodurch in vorteilhafter Weise ein besonders einfache, von der Drehposition des Verbindungsadapters unabhängige Medienübergabe möglich ist. Vorzugsweise ist die zentrisch angeordnete Öffnung an dem Ende des Verbindungsadapters vorgesehen, das zur Verbindung mit der ersten oder zweiten Aufbereitungs- und/ oder Pflegevorrichtung vorgesehen ist. Vorzugsweise ist eine Öffnung einer der mehreren Medienleitungen exzentrisch oder radial versetzt (in Bezug auf die Längsachse des Verbindungsadapters) angeordnet.

Vorzugsweise sind Öffnungen unterschiedlicher Medienleitungen, die an demselben Ende des Verbindungsadapters angeordnet sind, zum Beispiel an dem Ende des Verbindungsadapters zur Verbindung mit der ersten oder zweiten Aufbereitungs- und/ oder Pflegevorrichtung oder an dem Ende des Verbindungsadapters zur Verbindung mit dem medizinischen oder dentalen Instrument, an unterschiedlichen Oberflächen des Verbindungsadapters vorgesehen. Die unterschiedlichen Oberflächen sind zum Beispiel axial (in Bezug auf die Längsachse des Verbindungsadapters) versetzt angeordnet und/ oder durch Ringschultern mit unterschiedlichen Radien (von der Längsachse des Verbindungsadapters) gebildet. Damit wird in vorteilhafter Weise eine zuverlässige Medienübergabe erreicht, bei der zum Beispiel unterschiedliche Medien nicht miteinander in Kontakt kommen.

Vorzugsweise ist an dem Verbindungsadapter ein Anschlusselement zum Anschluss eines aufzubereitenden und/ oder zu pflegenden, medizinischen oder dentalen Instruments vorgesehen. Vorzugsweise ist das Anschlusselement an einem instrumentenseitigen Ende des Verbindungsadapters vorgesehen. Vorzugsweise umfasst das Anschlusselement ein Steckelement oder einen Teil einer Steckverbindung, wodurch in vorteilhafter Weise eine rasche und einfache Verbindung des Verbindungsadapters mit dem Instrument erreichbar ist. Vorzugsweise umfasst das Anschlusselement oder das Steckelement eine oder mehrere Öffnungen oder Sackbohrungen zur Aufnahme von Steckelementen des medizinischen oder dentalen Instruments, zum Beispiel von Leitungsabschnitten oder elektrischen Kontakten, die vom Kupplungsende des Instrumentes abstehen.

Vorzugsweise erstreckt sich die zumindest eine Medienleitung des Verbindungsadapters durch das Anschlusselement. Vorzugsweise endet die zumindest eine Medienleitung des Verbindungsadapters an dem Anschlusselement, insbesondere an einem freien Ende oder an einer Oberfläche des Anschlusselements, und/ oder ist die Öffnung der zumindest einen Medienleitung dort angeordnet, wodurch in vorteilhafter Weise eine einfache und sichere Medienübergabe an der Schnittstelle zwischen dem Verbindungsadapter und dem Instrument gebildet ist.

Vorzugsweise umfasst das Anschlusselement ein zylindrisches Rohr, das insbesondere einen Teil der Steckverbindung an der Schnittstelle zwischen dem Verbindungsadapter und dem medizinischen oder dentalen Instrument bildet. Vorzugsweise erstreckt sich die zumindest eine Medienleitung des Verbindungsadapters durch das zylindrische Rohr. Vorzugsweise endet die zumindest eine Medienleitung des Verbindungsadapters an dem zylindrischen Rohr, insbesondere an einem freien Ende oder an einer Oberfläche des zylindrischen Rohrs, und/ oder ist die Öffnung der zumindest einen Medienleitung dort angeordnet, wodurch in vorteilhafter Weise eine zuverlässige Medienübergabe an der Schnittstelle zwischen dem Verbindungsadapter und dem Instrument gebildet ist.

Besonders bevorzugt umfasst das Anschlusselement eine Überwurfmutter mit Innengewinde zur Schraubverbindung mit einem Gewinde, insbesondere einem Außengewinde, des medizinischen oder dentalen Instruments. Vorzugsweise ist die Überwurfmutter drehbar an dem Verbindungsadapter, insbesondere an dessen instrumentenseitigen Ende, besonders bevorzugt an dem zylindrischen Rohr angeordnet. Vorzugsweise sichert die Überwurfmutter die Steckverbindung des Verbindungsadapters mit dem medizinischen oder dentalen Instrument, so dass in vorteilhafter Weise eine ungewollte Trennung des Verbindungsadapters von dem Instrument verhindert ist.

Vorzugsweise ist die Überwurfmutter entlang einer Längsachse des Verbindungsadapters und/ oder des Anschlusselements und/ oder des zylindrischen Rohrs verschiebbar, wodurch in vorteilhafter Weise das Verbinden des Verbindungsadapters mit dem medizinischen oder dentalen Instrument erleichtert ist. Vorzugsweise ist das zylindrische Rohr zumindest teilweise in der Überwurfmutter aufgenommen. Vorzugsweise umgibt die Überwurfmutter zumindest einen Teil des zylindrischen Rohrs. Vorzugsweise ist die Überwurfmutter verschiebbar und drehbar an dem zylindrischen Rohr angeordnet.

Vorzugsweise umfasst das Anschlusselement, insbesondere das zylindrische Rohr, einen bewegbaren Fortsatz, zum Beispiel eine Nase, zum Eingriff in einen Rücksprung an dem medizinischen oder dentalen Instrument. Vorzugsweise sichert der Fortsatz die Steckverbindung des Verbindungsadapters mit dem Instrument, so dass in vorteilhafter Weise eine ungewollte Trennung des Verbindungsadapters von dem Instrument verhindert ist. Vorzugsweise ist der Fortsatz radial (in Bezug auf die Längsachse des Verbindungsadapters) bewegbar. Vorzugsweise ist der Fortsatz durch ein Federelement vorgespannt. Vorzugsweise ist der Fortsatz mit einem Bedienelement für einen Anwender verbunden, zum Beispiel mit einem Hebel, um insbesondere den Fortsatz radial zu bewegen, um damit die Verbindung zwischen dem Verbindungsadapter und dem Instrument herstellen und/ oder trennen zu können.

Vorzugsweise ist an dem Verbindungsadapter ein Kupplungselement zur (lösbaren) Verbindung des Verbindungsadapters mit einer oder mehreren Aufbereitungs- und/ oder Pflegevorrichtungen, insbesondere mit der im Vorstehenden beschriebenen ersten und zweiten Aufbereitungs- und/ oder Pflegevorrichtung, vorgesehen.

Vorzugsweise ist an zumindest einer Aufbereitungs- und/ oder Pflegevorrichtung, insbesondere an den im Vorstehenden beschriebenen ersten und zweiten Aufbereitungs- und/ oder Pflegevorrichtungen, ein Kupplungsstück zur (lösbaren) Verbindung mit einem, insbesondere im Vorstehenden beschriebenen, Verbindungsadapter, an den ein aufzubereitendes und/ oder zu pflegendes medizinisches oder dentales Instrument anschließbar ist, vorgesehen, so dass zwischen der zumindest einen Aufbereitungs- und/ oder Pflegevorrichtung und dem Verbindungsadapter und insbesondere einem daran angeschlossenen medizinische oder dentalen Instrument eine Fluid übertragende, insbesondere Aufbereitungs- und/ oder Pflegemedium übertragende, Verbindung herstellbar ist. Insbesondere umfasst das Kupplungsstück ein im Vorstehenden beschriebenes Kupplungsstück der erste oder zweite Aufbereitungs- und/ oder Pflegevorrichtung. Insbesondere ist das Kupplungsstück Teil einer Steckverbindung oder als Steckelement ausgebildet, so wie dies im Vorstehenden beschrieben ist.

Das Kupplungselement des Verbindungsadapters und das Kupplungsstück der zumindest einen Aufbereitungs- und/ oder Pflegevorrichtung bilden vorzugsweise eine Kupplungsvorrichtung, insbesondere eine Steckverbindung, die im Vorstehenden bereits beschrieben ist. Eines der beiden Bauteile, nämlich das Kupplungsstück oder das Kupplungselement, umfasst einen Kupplungsfortsatz und das andere der beiden Bauteile, nämlich das Kupplungsstück oder das Kupplungselement, umfasst eine Kupplungsaufnahme zur Steckaufnahme des Kupplungsfortsatzes.

Jenes der beiden Bauteile (Kupplungsstück oder Kupplungselement), das die Kupplungsaufnahme aufweist, bevorzugt das Kupplungsstück der zumindest einen Aufbereitungs- und/ oder Pflegevorrichtung, umfasst:
einen, vorzugsweise zylindrischen, Körper, in dem die Kupplungsaufnahme geformt ist;
zumindest eine Medienleitung zur Leitung eines Aufbereitungs- und/ oder Pflegemediums durch den Körper, wobei die Medienleitung in einer Öffnung an einer Oberfläche der Kupplungsaufnahme endet;
eine in dem Körper beweglich angeordnete Verriegelungsplatte, die wahlweise in eine Verriegelungsposition und eine entriegelte Position bewegbar ist;
eine in der Verriegelungsplatte angeordnete Durchgangsöffnung, wobei die Durchgangsöffnung mit der Öffnung der Medienleitung und/ oder vorzugsweise mit der Kupplungsaufnahme zum Durchtritt des Aufbereitungs- und/ oder Pflegemediums fluidverbunden ist;
ein erstes Vorspannelement zum Vorspannen der Verriegelungsplatte;
einen Verriegelungsstift, der verschiebbar in einer Bohrung des Körpers aufgenommen ist und ein Verriegelungsstück und ein Entriegelungsstück aufweist;
eine Verriegelungsöffnung in der Verriegelungsplatte, durch welche sich der Verriegelungsstift erstreckt, wobei die Verriegelungsöffnung einen Verriegelungsabschnitt und einen Entriegelungsabschnitt aufweist;
ein zweites Vorspannelement zum Vorspannen des Verriegelungsstifts derart, dass ein Kontaktende des Verriegelungsstifts über die Verriegelungsplatte hinausragt.

Im Folgenden wird das Zusammenwirken der genannten Komponenten des Bauteils mit der Kupplungsaufnahme, bevorzugt des Kupplungsstücks der zumindest einen Aufbereitungs- und/ oder Pflegevorrichtung, beschrieben, wobei sich die Verriegelungsplatte zu Beginn in ihrer entriegelten Position befindet. Dies dient ausschließlich der Veranschaulichung der Funktionsweise und stellt keine Einschränkung des erfindungsgemäßen Gegenstands dar.

Durch eine Krafteinwirkung an dem oder auf das Kontaktende des Verriegelungsstifts ist das Kontaktende entgegen der Vorspannung des zweiten Vorspannelements in Richtung der Bohrung des Körpers bewegbar. Durch die Krafteinwirkung und/ oder die Bewegung wird der Verriegelungsstift insbesondere weiter in Richtung der Bohrung und vorzugsweise zumindest ein Stück des Verriegelungsstifts in die Bohrung hineinbewegt oder verschoben. Durch die Bewegung ist oder wird das Verriegelungsstück des Verriegelungsstifts (selbsttätig) in dem Verriegelungsabschnitt der Verriegelungsöffnung aufgenommen, wodurch die Verriegelungsplatte aufgrund der Vorspannkraft des ersten Vorspannelements (aus der entriegelten Position) in die Verriegelungsposition bewegbar und in der Verriegelungsposition fixiert ist. Die Krafteinwirkung an dem oder auf das Kontaktende ist oder wird insbesondere durch das Einstecken oder Einschieben des Kupplungsfortsatzes in die Kupplungsaufnahme erzeugt, insbesondere durch Kontakt des Kupplungsfortsatzes oder eines Teils davon mit dem Kontaktende.

Durch eine Krafteinwirkung an der Verriegelungsplatte entgegen der Vorspannung des ersten Vorspannelements bewegt oder verschiebt sich die Verriegelungsplatte und nimmt wiederum die entriegelte Position ein, wodurch aufgrund der Vorspannkraft des zweiten Vorspannelements das Kontaktende des Verriegelungsstifts (selbsttätig) von der Bohrung des Körpers wegbewegbar ist oder sich wegbewegt, vorzugsweise wenn die Kupplungsaufnahme und der Kupplungsfortsatz und/ oder das Kupplungsstück und das Kupplungselement voneinander getrennt sind oder werden, so dass das Kontaktende ausreichend Platz hat sich von der Bohrung des Körpers wegzubewegen und/ oder die entriegelte Position einzunehmen. Insbesondere bewegt sich der Verriegelungsstift von der Bohrung weg und vorzugsweise bewegt sich zumindest ein Stück des Verriegelungsstifts aus der Bohrung hinaus. Durch diese Bewegung des Verriegelungsstifts verlässt das Verriegelungsstück des Verriegelungsstifts den Verriegelungsabschnitt der Verriegelungsöffnung und das Entriegelungsstück des Verriegelungsstifts wird oder ist wiederum in dem Entriegelungsabschnitt der Verriegelungsöffnung aufgenommen. Durch die Aufnahme des Entriegelungsstücks in dem Entriegelungsabschnitt ist die Verriegelungsplatte in der entriegelten Position fixiert.

Vorzugsweise sind in dem Körper zwei oder mehr Medienleitung zur Leitung jeweils eines Aufbereitungs- und/ oder Pflegemediums durch den Körper vorgesehen. Vorzugsweise endet jede Medienleitung in einer (eigenen) Öffnung an einer Oberfläche der Kupplungsaufnahme. Vorzugsweise ist jede Öffnung zur Übertragung eines Aufbereitungs- und/ oder Pflegemediums zwischen dem Verbindungsadapter, insbesondere dessen Kupplungselement, und der zumindest einen Aufbereitungs- und/ oder Pflegevorrichtung, insbesondere deren Kupplungsstück, vorgesehen. Mit diesen Ausgestaltungen ist in vorteilhafter Weise eine getrennte Übertragung der Medien möglich.

Vorzugsweise sind Öffnungen unterschiedlicher Medienleitungen, die sich an demselben Ende der Kupplungsaufnahme befinden, bevorzugt am selben Ende des Kupplungsstücks der zumindest einen Aufbereitungs- und/ oder Pflegevorrichtung, an unterschiedlichen Oberflächen der Kupplungsaufnahme vorgesehen. Die unterschiedlichen Oberflächen sind zum Beispiel axial (in Bezug auf die Längsachse der Kupplungsaufnahme) versetzt angeordnet und/ oder durch Ringschultern mit unterschiedlichen Radien (von der Längsachse der Kupplungsaufnahme) gebildet. Damit wird in vorteilhafter Weise eine zuverlässige Medienübergabe erreicht, bei der unterschiedliche Medien nicht miteinander in Kontakt kommen.

Vorzugsweise ist die zumindest eine Öffnung oder eine der mehreren Öffnungen der mehreren Medienleitungen zentrisch (in Bezug auf die Längsachse der Kupplungsaufnahme) angeordnet, wodurch in vorteilhafter Weise ein besonders einfache, von der Drehposition der Kupplungsaufnahme unabhängige Medienübergabe möglich ist. Vorzugsweise ist die zentrisch angeordnete Öffnung gegenüber einer zentrischen Öffnung des Kupplungsfortsatzes und/ oder des Verbindungsadapters anordenbar (wenn der Kupplungsfortsatz und die Kupplungsaufnahme miteinander verbunden sind).

Vorzugsweise ist die zumindest eine Öffnung oder eine der mehreren Öffnungen der mehreren Medienleitungen exzentrisch oder radial versetzt (in Bezug auf die Längsachse der Kupplungsaufnahme) angeordnet.

Vorzugsweise ist die Verriegelungsplatte und/ oder die Durchgangsöffnung quer oder orthogonal zu der zumindest einen Medienleitung in dem Körper und/ oder zur Längsachse der Kupplungsaufnahme beweglich oder verschiebbar. Vorzugsweise ist die Verriegelungsplatte als längliche und/ oder im Wesentlichen rechteckige Platte ausgebildet.

Vorzugsweise ist eine Nut in dem Körper vorgesehen, in welcher die Verriegelungsplatte und/ oder die Durchgangsöffnung beweglich oder verschiebbar aufgenommen und insbesondere geführt ist. Vorzugsweise sind die Ränder der Verriegelungsplatte in der Nut, insbesondere in Führungen der Nut aufgenommen. Vorzugsweise ist die Nut derart ausgebildet und/ oder die Verriegelungsplatte derart darin aufgenommen, dass die Durchgangsöffnung frei zugänglich ist, insbesondere zum Einstecken des Kupplungsfortsatzes. Vorzugsweise ist die Nut quer oder orthogonal zu der zumindest einen Medienleitung in dem Körper und/ oder zur Längsachse der Kupplungsaufnahme angeordnet. Vorzugsweise ist die Nut an einem Ende der Kupplungsaufnahme vorgesehen, insbesondere an einem dem Kupplungsfortsatz zugewandten Ende (wenn der Kupplungsfortsatz und die Kupplungsaufnahme miteinander verbunden sind). Damit ist eine zuverlässige Bewegung und Führung der Verriegelungsplatte und/ oder der Durchgangsöffnung gewährleistet.

Die Verriegelungsplatte und/ oder insbesondere die Durchgangsöffnung sind wahlweise in eine Verriegelungsposition und eine entriegelte Position bewegbar. In der Verriegelungsposition sind die Kupplungsaufnahme und der Kupplungsfortsatz und/ oder das Kupplungsstück und das Kupplungselement und/ oder der Verbindungsadapter und die Aufbereitungs- und/ oder Pflegevorrichtung miteinander verbunden, insbesondere Fluid oder Aufbereitungs- und/ oder Pflegemedium übertragend verbunden. In der Verriegelungsposition greifen oder stecken die Kupplungsaufnahme und der Kupplungsfortsatz vorzugsweise ineinander, insbesondere ist der Kupplungsfortsatz in der Kupplungsaufnahme aufgenommen.

In der entriegelten Position sind die Kupplungsaufnahme und der Kupplungsfortsatz und/ oder das Kupplungsstück und das Kupplungselement und/ oder der Verbindungsadapter und die Aufbereitungs- und/ oder Pflegevorrichtung voneinander getrennt oder trennbar. In der entriegelten Position greifen oder stecken die Kupplungsaufnahme und der Kupplungsfortsatz vorzugsweise nicht ineinander, insbesondere ist der Kupplungsfortsatz nicht in der Kupplungsaufnahme aufgenommen.

Vorzugsweise ist die in der Verriegelungsplatte angeordnete Durchgangsöffnung derart bemessen, dass zumindest ein Teil des Kupplungsfortsatzes darin aufnehmbar ist und/ oder durchragen kann. Vorzugsweise ist die Durchgangsöffnung durch eine Umfangskante begrenzt oder definiert die Umfangskante in der Verriegelungsplatte. Die Umfangskante ist dazu vorgesehen, in einen Rücksprung an dem Kupplungsfortsatz einzugreifen, wenn der Kupplungsfortsatz in die Kupplungsaufnahme eingesteckt ist und die Verriegelungsplatte die Verriegelungsposition einnimmt, so dass das Kupplungsstück und das Kupplungselement aneinander befestigt sind. Damit ist eine zuverlässige Verbindung zwischen der Kupplungsaufnahme und dem Kupplungsfortsatz herstellbar.

Das erste Vorspannelement zum Vorspannen der Verriegelungsplatte und das zweite Vorspannelement zum Vorspannen des Verriegelungsstifts sind vorzugsweise als Federelemente, zum Beispiel als Spiralfedern, Druck- oder Zugfedern ausgebildet.

Der Verriegelungsstift ist vorzugsweise quer oder orthogonal zur Verriegelungsplatte und/ oder im Wesentlichen parallel zur zumindest einen Medienleitung oder zur Längsachse der Kupplungsaufnahme bewegbar oder verschiebbar, wodurch in vorteilhafter Weise eine einfache Betätigung des Verriegelungsstifts möglich ist.

Der verschiebbar in einer Bohrung des Körpers aufgenommene, insbesondere längliche Verriegelungsstift umfasst ein Verriegelungsstück, ein Entriegelungsstück und ein Kontaktende. Der Außendurchmesser des Verriegelungsstücks ist insbesondere geringer ist als der Außendurchmesser des Entriegelungsstücks. Das Kontaktende ist vorzugsweise an einem (freien) Ende des Verriegelungsstifts angeordnet und/ oder bildet ein freies Ende des Verriegelungsstifts. Das Kontaktende umfasst insbesondere eine ebene Kontaktfläche. Das Verriegelungsstück, das Entriegelungsstück und das Kontaktende sind vorzugsweise axial (bezogen auf eine Längsachse des Kontaktstiftes) angeordnet. Besonders bevorzugt ist das Verriegelungsstück zwischen dem Entriegelungsstück und dem Kontaktende angeordnet. Durch diesen Aufbau des Verriegelungsstifts ist eine zuverlässige Funktion der Kupplungsvorrichtung gewährleistet.

In der Verriegelungsplatte ist eine Verriegelungsöffnung vorgesehen, durch welche sich der Verriegelungsstift erstreckt. Zumindest das Kontaktende des Verriegelungsstifts, vorzugsweise zumindest auch ein Teil des Verriegelungsstücks, ragt über die Verriegelungsplatte hinaus, so dass insbesondere das Kontaktende (für ein Kraft ausübendes Element) frei zugänglich ist, wodurch in vorteilhafter Weise eine einfache Betätigung des Verriegelungsstifts möglich ist, insbesondere durch Kontakt durch den Kupplungsfortsatz, so wie dies im Vorstehenden bereits beschrieben ist.

Die Verriegelungsöffnung weist vorzugsweise eine längliche Form und/ oder Schlitzform auf. Die Verriegelungsöffnung ist vorzugsweise benachbart zur Durchgangsöffnung angeordnet. Die Verriegelungsöffnung ist vorzugsweise in derselben Ebene wie die Durchgangsöffnung angeordnet. Die Verriegelungsöffnung ist zum Beispiel durch einen, insbesondere stegförmigen, Abschnitt der Verriegelungsplatte von der Durchgangsöffnung getrennt. Alternativ mündet die Verriegelungsöffnung in die Durchgangsöffnung, so dass die Verriegelungsöffnung insbesondere einen schlitzförmigen Fortsatz der Durchgangsöffnung bildet. Durch diesen Aufbau der Verriegelungsöffnung ist eine zuverlässige Funktion der Kupplungsvorrichtung gewährleistet.

Die Verriegelungsöffnung weist einen Verriegelungsabschnitt, insbesondere zur Aufnahme des Verriegelungsstücks des Verriegelungsstifts, und einen Entriegelungsabschnitt, insbesondere zur Aufnahme des Entriegelungsstücks des Verriegelungsstifts auf. Vorzugsweise ist der Innendurchmesser des Verriegelungsabschnitts geringer als der Innendurchmesser des Entriegelungsabschnitts. Vorzugsweise ist der Entriegelungsabschnitt näher zur Durchgangsöffnung angeordnet und der Verriegelungsabschnitt von der Durchgangsöffnung weiter entfernt. Durch diesen Aufbau der Verriegelungsöffnung ist eine zuverlässige Interaktion mit dem Verriegelungsstift gewährleistet.

Vorzugsweise ist an der Verriegelungsplatte ein Stellelement vorgesehen, wodurch in vorteilhafter Weise das Betätigen der Verriegelungsplatte, insbesondere, wenn diese von der Verriegelungsposition in die entriegelte Position bewegt wird, für den Anwender erleichtert wird. Bevorzugt ist das Stellelement im Wesentlichen gegenüber der Verriegelungsöffnung angeordnet. Vorzugsweise ist das Stellelemente ein Teil der Verriegelungsplatte, insbesondere ein integral oder einteilig ausgebildeter Teil der Verriegelungsplatte. Alternativ oder zusätzlich ist das Stellelement vorzugsweise plattenförmig geformt. Alternativ oder zusätzlich ist das Stellelement vorzugsweise gewinkelt zum dem Abschnitt der Verriegelungsplatte, in dem sich die Durchgangsöffnung und/ oder die Verriegelungsöffnung befinden, angeordnet, insbesondere in etwa um 90° gewinkelt. Alternativ oder zusätzlich ist das Stellelement vorzugsweise in etwa parallel zur Kupplungsaufnahme angeordnet.

Das andere der beiden Bauteile (Kupplungsstück oder Kupplungselement), bevorzugt das Kupplungselement des Verbindungsadapters, umfasst den Kupplungsfortsatz zur Aufnahme in der Kupplungsaufnahme. Der Kupplungsfortsatz ist vorzugsweise zylindrisch geformt. Der Kupplungsfortsatz weist vorzugsweise mehrere Abschnitte mit unterschiedlichen Außendurchmessern auf.

Vorzugsweise erstreckt sich zumindest eine Medienleitung zur Leitung eines Aufbereitungs- und/ oder Pflegemediums durch den Kupplungsfortsatz. Insbesondere ist die zumindest eine Medienleitung mit der Medienleitung des Verbindungsadapters verbunden oder ist Teil der Medienleitung des Verbindungsadapters. Vorzugsweise ist die zumindest eine Medienleitung mit einer Öffnung an einer Oberfläche des Kupplungsfortsatzes verbunden. Vorzugsweise ist die Öffnung zentrisch (in Bezug auf die Längsachse des Kupplungsfortsatzes und/ oder des Verbindungsadapters) angeordnet, so dass sie insbesondere in Fluidverbindung mit der zentrischen Öffnung der Kupplungsaufnahme steht, wodurch in vorteilhafter Weise eine zuverlässige Medienübergabe an der Schnittstelle zwischen dem Verbindungsadapter und der Aufbereitungs- und/ oder Pflegevorrichtung gebildet ist.

Vorzugsweise ist an dem Kupplungsfortsatz ein Rücksprung vorgesehen, in den die Umfangskante der Durchgangsöffnung der Verriegelungsplatte eingreift, wenn der Kupplungsfortsatz in die Kupplungsaufnahme eingesteckt ist und die Verriegelungsplatte die Verriegelungsposition einnimmt, wodurch das Kupplungsstück und das Kupplungselement in vorteilhafter Weise zuverlässig aneinander befestigt sind. Der Rücksprung umfasst zum Beispiel eine Nut, eine Ringnut, eine Aussparung oder ähnliche Elemente. Um die Kupplungsaufnahme und den Kupplungsfortsatz und/ oder das Kupplungsstück und das Kupplungselement voneinander trennen zu können, muss der Eingriff der Umfangskante in den Rücksprung beendet werden, was durch das Verschieben der Verriegelungsplatte entgegen der Federkraft des ersten Vorspannelements bewirkbar ist, so wie dies im Vorstehenden bereits beschrieben ist.

Vorzugsweise ist an dem Kupplungsfortsatz eine Kontaktfläche vorgesehen, die derart angeordnet ist, dass sie beim Einstecken des Kupplungsfortsatzes in die Kupplungsaufnahme durch Kontakt mit dem Kontaktende die Krafteinwirkung auf das Kontaktende des Verriegelungsstifts bewirkt, um das Kontaktende entgegen der Vorspannung des zweiten Vorspannelements in Richtung der Bohrung des Körpers zu bewegen, bis das Verriegelungsstück des Verriegelungsstifts in dem Verriegelungsabschnitt der Verriegelungsöffnung aufgenommen ist, wodurch die Verriegelungsplatte aufgrund der Vorspannkraft des ersten Vorspannelements in die Verriegelungsposition bewegt wird. Dadurch ist in vorteilhafter Weise die Verriegelung zwischen dem Kupplungsfortsatz und der Kupplungsaufnahme durch das Einschieben des Kupplungsfortsatzes in die Kupplungsaufnahme bewirkt, ohne zusätzlich notwendige Handgriffe durch den Anwender. Die Kontaktfläche umfasst zum Beispiel eine Ringfläche, Ringschulter und/ oder eine Stufe an dem Kupplungsfortsatz.

Die im Vorstehenden beschriebene Kupplungsvorrichtung kann in einem im Vorstehenden beschriebenen Verfahren zur Aufbereitung und/ oder Pflege eines medizinischen oder dentalen Instruments oder in einem im Vorstehenden beschriebenen System zur Aufbereitung und/ oder Pflege eines medizinischen oder dentalen Instruments Verwendung finden. Alternativ ist die Kupplungsvorrichtung eine eigenständige Erfindung, die in beliebigen anderen Verfahren zur Aufbereitung und/ oder Pflege eines medizinischen oder dentalen Instruments, Aufbereitungs- und/ oder Pflegevorrichtungen für medizinische oder dentale Instrumente oder Systemen zur Aufbereitung und/ oder Pflege eines medizinischen oder dentalen Instruments implementiert sein kann.

Vorzugsweise ist eine Aufbereitungs- und/ oder Pflegevorrichtung zur Aufbereitung und/ oder Pflege eines medizinischen oder dentalen Instruments vorgesehen, die ein Kupplungsstück mit einer im Vorstehenden beschriebenen Kupplungsaufnahme aufweist. Besonders bevorzugt umfasst die Aufbereitungs- und/ oder Pflegevorrichtung die im Vorstehenden beschriebene Kupplungsvorrichtung und den im Vorstehenden beschriebenen Verbindungsadapter.

Vorzugsweise umfasst die Aufbereitungs- und/ oder Pflegevorrichtung zumindest eines der folgenden Geräte: ein Pflegegerät, das insbesondere ein Pflegemedium, vorzugsweise wie im Vorstehenden beschrieben, an das medizinische oder dentale Instrument abgibt; ein Aufbereitungs- oder Reinigungsgerät, das insbesondere ein Aufbereitungs- oder Reinigungsmedium, vorzugsweise wie im Vorstehenden beschrieben, an das medizinische oder dentale Instrument abgibt; ein Desinfektionsgerät das insbesondere ein Desinfektionsmedium, vorzugsweise wie im Vorstehenden beschrieben, an das medizinische oder dentale Instrument abgibt; einen Sterilisator oder Autoklaven, der ein Sterilisationsmedium, vorzugsweise wie im Vorstehenden beschrieben, an das medizinische oder dentale Instrument abgibt.

Vorzugsweise umfasst die Aufbereitungs- und/ oder Pflegevorrichtungen zumindest eine Medienquelle und/ oder Medienzuführung für Betriebsmedien und/oder Aufbereitungs- und/ oder Pflegemedien. Die zwei unterschiedlichen Aufbereitungs- und/ oder Pflegevorrichtungen umfassen insbesondere (räumlich) getrennte Kammern zur Aufnahme des aufzubereitenden und/ oder zu pflegenden medizinischen oder dentalen Instruments, jeweils mit zumindest einem Kupplungsstück zum Anschluss des Instruments, so dass es zur Durchführung des Verfahrens und/ oder der zumindest zwei Behandlungen notwendig ist, das Instrument (zeitlich versetzt) in die Kammer der ersten Aufbereitungs- und/ oder Pflegevorrichtung und der zweiten Aufbereitungs- und/ oder Pflegevorrichtung einzubringen und insbesondere das Instrument mit dem Kupplungsstück der jeweiligen Kammer zu verbinden.

Im Übrigen kann die Aufbereitungs- und/ oder Pflegevorrichtung eines oder mehrere Merkmale der im Vorstehenden beschriebenen Aufbereitungs- und/ oder Pflegevorrichtungen aufweisen, so dass zur Vermeidung von Wiederholungen auf diese Merkmale verwiesen wird.

Die Erfindung wird nachfolgend anhand bevorzugter Ausführungsbeispiele und Bezug nehmend auf die beigefügten Zeichnungen erläutert:

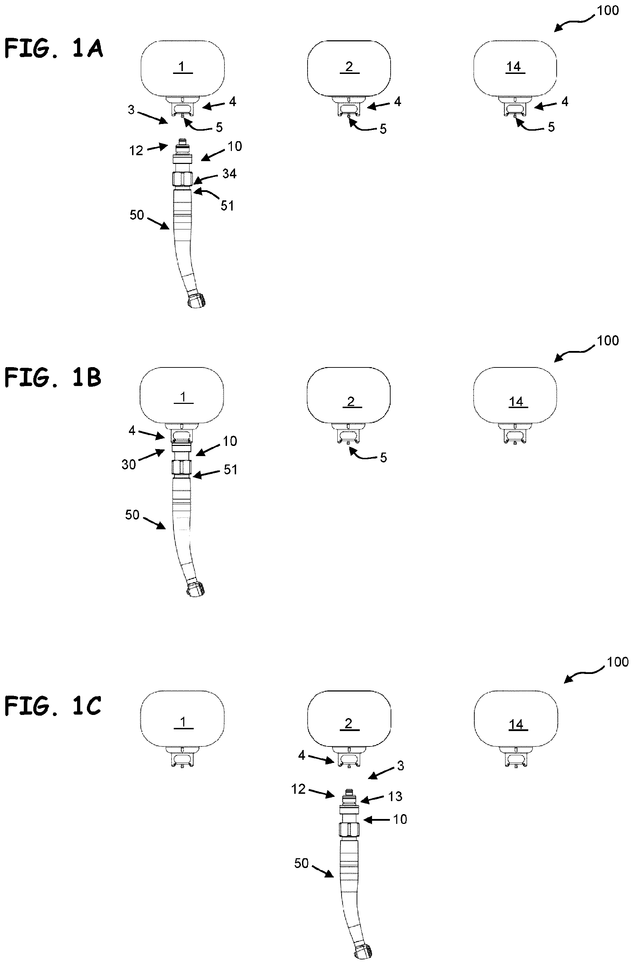

Die Figuren 1A - 1F zeigen ein System zur Aufbereitung und/ oder Pflege eines medizinischen oder dentalen Instruments und einen schrittweisen Ablauf eines Verfahrens zur Aufbereitung und/ oder Pflege eines medizinischen oder dentalen Instruments mit drei unterschiedlichen Aufbereitungs- und/ oder Pflegevorrichtungen, wobei ein medizinisches oder dentales Instrument und ein damit lösbar verbundener Verbindungsadapter gemeinsam und zeitlich aufeinander folgend an jedes der drei unterschiedlichen Aufbereitungs- und/ oder Pflegevorrichtungen angeschlossen wird, so dass das medizinische oder dentale Instrument durch die drei Aufbereitungs- und/ oder Pflegevorrichtungen aufbereitet und/ oder gepflegt werden kann;
Die Figuren 2A - 2C zeigen einen Verbindungsadapter, der lösbar mit einem medizinischen oder dentalen Instrument verbindbar ist und der dazu vorgesehen ist, das medizinische oder dentale Instrument mit einer oder mehrere Aufbereitungs- und/ oder Pflegevorrichtungen zu verbinden, so dass das medizinische oder dentale Instrument durch die drei Aufbereitungs- und/ oder Pflegevorrichtungen aufbereitet und/ oder gepflegt werden kann;
Die Figuren 3A und 3B zeigen ein erstes medizinisches oder dentales Instrument und einen damit lösbar verbindbaren oder verbundenen Verbindungsadapter;
Die Figuren 4A und 4B zeigen ein zweites medizinisches oder dentales Instrument mit einer Drehkupplung und einen damit lösbar verbindbaren oder verbundenen Verbindungsadapter;
Figur 5 zeigt ein Ausführungsbeispiel einer Aufbereitungs- und/ oder Pflegevorrichtung in Form eines Pflegegeräts, in dessen Pflegekammer ein medizinisches oder dentales Instrument aufgenommen ist, das durch einen Verbindungsadapter mit einem Kupplungsstück des Pflegegeräts lösbar verbunden ist, so dass ein Pflegemedium von dem Pflegegerät in das Instrument übertragbar ist;
Figur 6 zeigt ein Ausführungsbeispiel eines Kupplungsstücks einer Aufbereitungs- und/ oder Pflegevorrichtung, das insbesondere zur lösbaren Verbindung mit einem medizinischen oder dentalen Instrument mittels eines Verbindungsadapters vorgesehen ist;
Figur 7 zeigt eine Schnittansicht durch das Kupplungsstück der Figur 6;
Figur 8 zeigt eine Detailansicht des Verriegelungsbereichs des Kupplungsstücks der Figur 6.

Das in den Figuren 1A - 1F dargestellte System 100 zur Aufbereitung und/ oder Pflege eines medizinischen oder dentalen Instruments 50 umfasst mehrere, hier drei, unterschiedliche Aufbereitungs- und/ oder Pflegevorrichtungen 1, 2, 14, die unterschiedliche Aufbereitungs- und/ oder Pflegeprozesse an dem Instrument 50 anwenden. Beispielsweise kann die Aufbereitungs- und/ oder Pflegevorrichtung 1 ein Reinigungs- und/ oder Desinfektionsgerät aufweisen, das insbesondere dazu vorgesehen ist, das Instrument 50 mit einem Desinfektionsmittel und/ oder mit einem Reinigungsmittel und/ oder mit heißem Wasser zu behandeln; beispielsweise kann die Aufbereitungs- und/ oder Pflegevorrichtung 2 ein Pflegegerät aufweisen, welches insbesondere dazu vorgesehen ist, das Instrument 50 mit einem Druckgas (Druckluft) durchzublasen und/ oder mit einem Schmiermittel zu versorgen; beispielsweise kann die Aufbereitungs- und/ oder Pflegevorrichtung 14 einen Sterilisator oder Autoklaven zur Abgabe eines Sterilisationsmediums an das Instrument 50 aufweisen.

Es wird ausdrücklich noch einmal darauf hingewiesen, dass das in den Figuren 1A - 1F dargestellte System 100 sowie die genannten Aufbereitungs- und/ oder Pflegevorrichtungen 1, 2, 14 beispielhaft sind. So kann insbesondere die Anzahl der mehreren Aufbereitungs- und/ oder Pflegevorrichtungen 1, 2, 14 variieren. Es ist zum Beispiel auch möglich, dass das System 100 nur zwei Aufbereitungs- und/ oder Pflegevorrichtungen aufweist, vorzugsweise ein Pflegegerät 2, welches insbesondere dazu vorgesehen ist, das Instrument 50 mit einem Druckgas (Druckluft) durchzublasen und/ oder mit einem Schmiermittel zu versorgen, und einen Sterilisator oder Autoklaven 14 zur Abgabe eines Sterilisationsmediums an das Instrument 50. Auch können die Geräte, welche das System 100 bilden, variieren, so kann zum Beispiel auch ein Gerät, das zur Aufbereitung und/ oder Pflege des Instruments 50 ein anderes als die genannten Aufbereitungs- und/ oder Pflegemedien oder eine andere Technologie, zum Beispiel Ultraschall oder eine Strahlung, etwa UV-Strahlung, anwendet, Teil des Systems 100 sein.

Die in den Figuren 1A - 1F schematisch dargestellten Aufbereitungs- und/ oder Pflegevorrichtungen 1, 2, 14 umfassen jeweils zumindest ein Kupplungsstück 4 mit einer Kupplungsaufnahme 5 zum Anschluss des aufzubereitenden und/ oder zu pflegenden Instruments 50. Das Kupplungsstück 4 ist als Teil einer Steckverbindung 3 ausgebildet. Das Kupplungsstück 4 oder die Kupplungsaufnahme 5 ist insbesondere fluidübertragend ausgebildet, so dass zumindest ein Aufbereitungs- und/ oder Pflegemedium über das Kupplungsstück 4 an das medizinische oder dentale Instrument 50 übertragbar ist. Besonders bevorzugt weist das Kupplungsstück 4 zumindest eine Fluidleitung auf, die derart in einer Öffnung an einer Oberfläche des Kupplungsstücks 4 endet, dass das in der Fluidleitung förderbare Aufbereitungs- und/ oder Pflegemedium in das Innere des Instruments 50 übertragbar ist.

In den Figuren 1A - 1F ist des Weiteren ein aufzubereitendes und/ oder zu pflegendes, medizinisches oder dentales Instrument 50 zu sehen, welches hier beispielhaft als dentales Handstück ausgebildet ist. An dem Kupplungsende 51 des Instruments 50 ist lösbar ein Verbindungsadapter 10 befestigt. Der Verbindungsadapter 10 weist dazu ein Anschlusselement 34 auf, das mit dem Kupplungsende 51 des medizinischen oder dentalen Instruments 50 oder mit einem Kupplungsende einer mit dem Instrument 50 lösbar verbundenen Drehkupplung verbindbar ist, insbesondere fluidübertragend verbindbar, so dass ein Aufbereitungs- und/ oder Pflegemedium in das Innere des Instruments 50 übertragbar ist.

An dem Verbindungsadapter 10 ist des Weiteren ein Kupplungselement 12 vorgesehen, insbesondere in Form eines Kupplungsfortsatzes 13, der mit dem Kupplungsstück 4 der Aufbereitungs- und/ oder Pflegevorrichtungen 1, 2, 14 lösbar verbindbar ist. Das Kupplungselement 12 und/ oder der Kupplungsfortsatz 13 bilden insbesondere einen Teil der Steckverbindung 3, bevorzugt ist der Kupplungsfortsatz 13 in das Kupplungsstück 4 einsteckbar. Die Verbindung zwischen dem Kupplungselement 12 und dem Kupplungsstück 4 ist vorzugsweise wiederum fluidübertragend, so dass ein Aufbereitungs- und/ oder Pflegemedium in das Innere des Instruments 50 übertragbar ist.

Die Kupplungsstücke 4 der Aufbereitungs- und/ oder Pflegevorrichtungen 1, 2, 14 sind derart ausgebildet, dass der Verbindungsadapter 10 an jedes Kupplungsstück 4 (funktionsgemäß oder funktionsfähig) anschließbar ist, insbesondere derart dass zumindest ein Aufbereitungs- und/ oder Pflegemedium von einer Aufbereitungs- und/ oder Pflegevorrichtung 1, 2, 14 über den Verbindungsadapter 10 in das Innere des Instruments 50 übertragbar ist. Vorzugsweise sind die Kupplungsstücke 4 der mehreren Aufbereitungs- und/ oder Pflegevorrichtungen 1, 2, 14 ident oder im Wesentlichen ident ausgebildet.

Ein Verfahren zur Aufbereitung und/ oder Pflege eines medizinischen oder dentalen Instruments 50, das mit dem System 100 durchführbar ist, ist somit dadurch definiert, dass das Instrument 50 und der damit verbundene Verbindungsadapter 10 gemeinsam oder als eine Einheit, insbesondere als eine für das Verfahren zur Aufbereitung und/ oder Pflege temporär gebildete Einheit, das Verfahren durchlaufen. D.h. das Instrument 50 und der Verbindungsadapter 10 werden als Einheit an zumindest zwei Geräte der Aufbereitungs- und/ oder Pflegevorrichtungen 1, 2, 14 angeschlossen und das Instrument 50 durch die jeweilige Aufbereitungs- und/ oder Pflegevorrichtung 1, 2, 14 aufbereitet oder gepflegt, ohne dass das Instrument 50 und der Verbindungsadapter 10 voneinander getrennt werden.

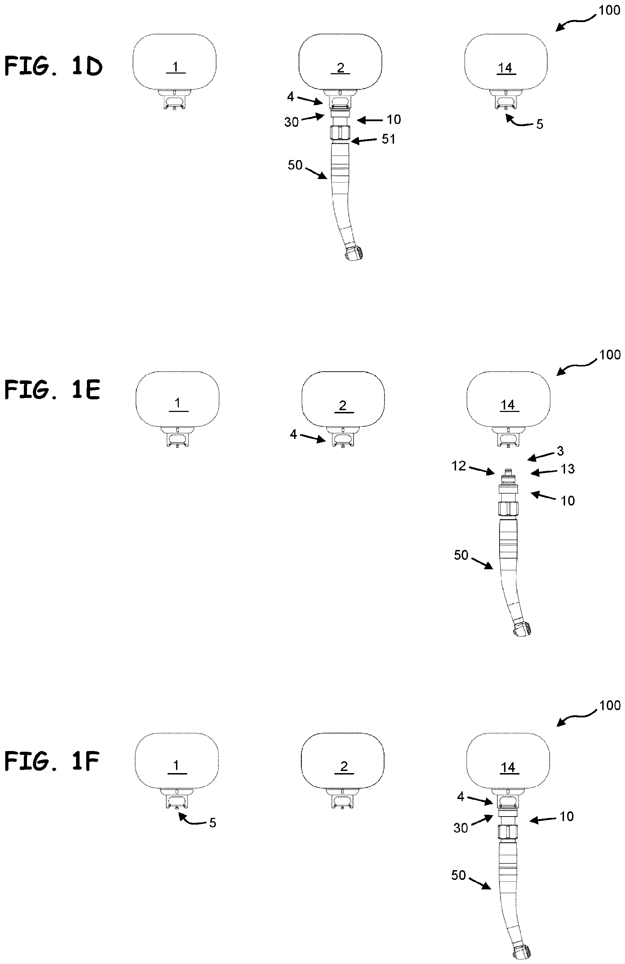

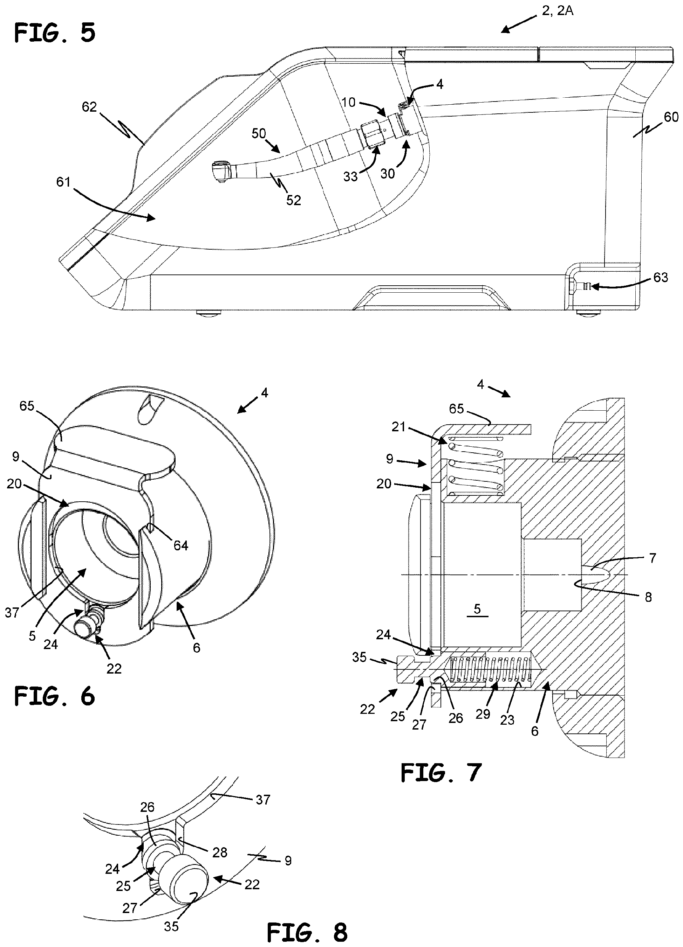

In Figur 1A ist das vor dem Beginn der ersten Aufbereitung und/ oder Pflege durch die Aufbereitungs- und/ oder Pflegevorrichtung 1 mit dem Verbindungsadapter 10 verbundene Instrument 50 zu erkennen. In Figur 1B sind das Instrument 50 und der Verbindungsadapter 10 an die erste Aufbereitungs- und/ oder Pflegevorrichtung 1 angeschlossen, so dass die Aufbereitung und/ oder Pflege, insbesondere auch des Inneren des Instruments 50, beginnen kann oder läuft. Nach Abschluss der Aufbereitung und/ oder Pflege durch die Aufbereitungs- und/ oder Pflegevorrichtung 1 werden das Instrument 50 und der Verbindungsadapter 10 gemeinsam (als Einheit) von der Aufbereitungs- und/ oder Pflegevorrichtung 1 gelöst, siehe Figur 1C, und anschließend gemeinsam (als Einheit) an die Aufbereitungs- und/ oder Pflegevorrichtung 2 gesteckt, siehe Figur 1D und auch Figur 5. Es folgt eine Aufbereitung und/ oder Pflege, insbesondere auch des Inneren des Instruments 50, durch die Aufbereitungs- und/ oder Pflegevorrichtung 2, nach deren Beendigung das Instrument 50 und der Verbindungsadapter 10 gemeinsam (als Einheit) von der Aufbereitungs- und/ oder Pflegevorrichtung 2 getrennt werden, siehe Figur 1E. Abschließend werden das Instrument 50 und der Verbindungsadapter 10 gemeinsam (als Einheit) an die Aufbereitungs- und/ oder Pflegevorrichtung 14 der Figur 1F angeschlossen, um das Instrument 50, insbesondere auch dessen Inneres, aufzubereiten und/ oder zu pflegen. Nach Abschluss der letzten Aufbereitung und/ oder Pflege werden das Instrument 50, der Verbindungsadapter 10 und die Aufbereitungs- und/ oder Pflegevorrichtung 14 voneinander getrennt, wobei vorzugsweise zuerst das Instrument 50 und der Verbindungsadapter 10 gemeinsam (als Einheit) von der Aufbereitungs- und/ oder Pflegevorrichtung 14 gelöst werden und anschließend der Verbindungsadapter 10 von dem Instrument 50 abgenommen wird.

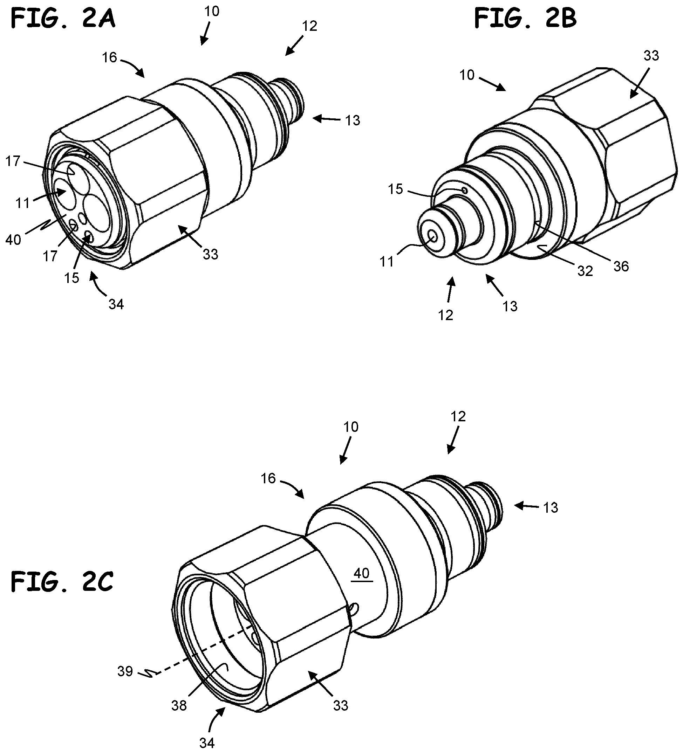

In den Figuren 2A - 8 werden insbesondere ein bevorzugtes Ausführungsbeispiel eines Verbindungsadapters 10 und einer Kupplungsvorrichtung 30 zur Verbindung des Verbindungsadapters 10 mit einer Aufbereitungs- und/ oder Pflegevorrichtung 1, 2, 14 dargestellt und beschrieben. Der Verbindungsadapter 10 und die Kupplungsvorrichtung 30 der Figuren 2A - 8 können in dem im Vorstehenden System 100 und Verfahren zur Aufbereitung und/ oder Pflege eines medizinischen oder dentalen Instruments 50 verwendet werden, was aufgrund der einfachen Handhabung zum Verbinden und Lösen des Verbindungsadapters 10 und der Kupplungsvorrichtung 30 sicherlich äußerst vorteilhaft ist. Grundsätzlich ist es jedoch auch möglich, beliebige andere Kupplungsvorrichtung und/ oder Verbindungsadapter für das System 100 und insbesondere das Verfahren zur Aufbereitung und/ oder Pflege mit dem System 100 einzusetzen. Umgekehrt können der Verbindungsadapter 10 und die Kupplungsvorrichtung 30 bei jeder beliebigen Aufbereitungs- und/ oder Pflegevorrichtung verwendet werden, die insbesondere nicht Teil eines Systems 100 von mehreren Aufbereitungs- und/ oder Pflegevorrichtung ist und/ oder die nicht in einem Verfahren zur Aufbereitung und/ oder Pflege eingesetzt wird, bei dem das aufzubereitende und/ oder zu pflegende Instrument 50 gemeinsam mit dem Verbindungsadapter 10 als Einheit an zumindest zwei Aufbereitungs- und/ oder Pflegevorrichtung angeschlossen wird.

Der in den Figuren 2A- 2C dargestellte Verbindungsadapter 10 umfasst einen, insbesondere länglichen, Körper 16, der sich entlang einer Längsachse 39 erstreckt. An einem Ende des Körpers 16 ein Anschlusselement 34 zum lösbaren Anschluss eines aufzubereitenden und/ oder zu pflegenden, medizinischen oder dentalen Instruments 50, 50A, 50B vorgesehen. An einem gegenüberliegenden Ende des Körpers 16 befindet sich ein Kupplungselement 12 zur lösbaren Verbindung mit einem Kupplungsstück 4 einer Aufbereitungs- und/ oder Pflegevorrichtung 1, 2, 14.

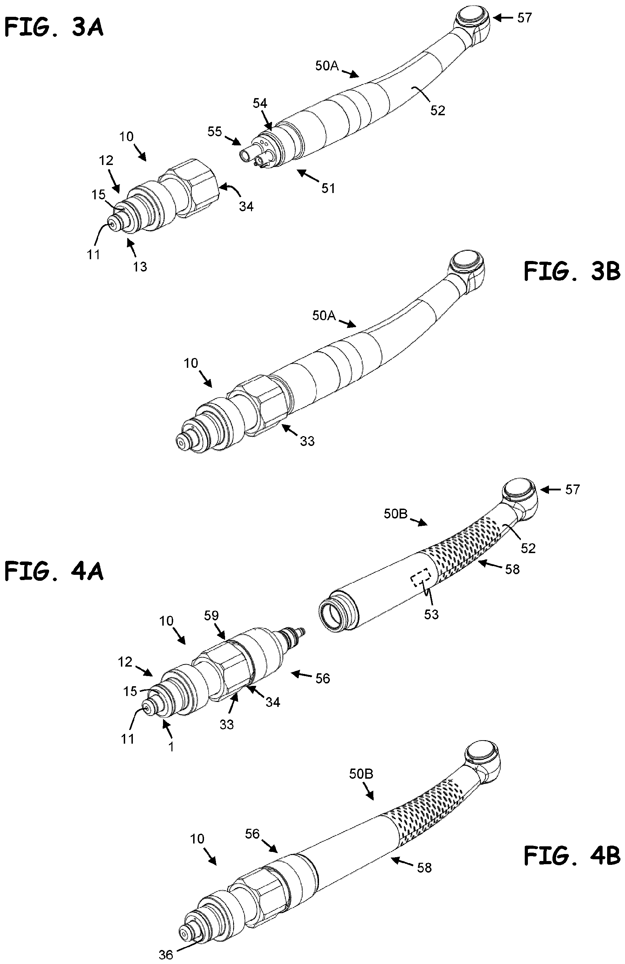

Zur Verbindung mit dem Instrument 50, 50A, 50B ist das Anschlusselement 34 als ein Steckelement oder Teil einer Steckverbindung ausgebildet. Insbesondere umfasst das Anschlusselement 34 zumindest eine oder mehrere Öffnungen 17 zur Aufnahme eines Fortsatzes 55 am Kupplungsende 51 des Instruments 50, 50A, 50B (siehe Figur 3A) oder an einer Drehkupplung 56 (siehe Figur 3B), zum Beispiel eines Abschnitts einer durch das Instrument 50, 50A, 50B verlaufenden Medienleitung und/ oder eines elektrischen Kontakts.

An dem Anschlusselement 34 oder als Teil des Anschlusselements 34 ist ein zylindrisches Rohr 40 vorgesehen. Die zumindest eine Öffnung 17 ist an einer freien Endfläche des zylindrischen Rohrs 40 angeordnet. An dem Anschlusselement 34 oder als Teil davon ist des Weiteren eine Überwurfmutter 33 mit Innengewinde 38 zur Schraubverbindung mit einem Außengewinde 54 (siehe Figur 3A) des medizinischen oder dentalen Instruments 50, 50 A, 50B oder der Drehkupplung 56 (siehe Figur 3B) vorgesehen. Die Überwurfmutter 33 ist drehbar an dem Verbindungsadapter 10, insbesondere an dessen zylindrischen Rohr 40, angeordnet. Nach dem Anstecken des Verbindungsadapters 10 an das Instruments 50, 50 A, 50B über die zumindest eine Öffnung 17 und den zumindest einen Fortsatz 55 werden die beiden Gewinde 38, 54 zur Sicherung dieser Steckverbindung miteinander verschraubt. Um das Verbinden des Verbindungsadapters 10 mit dem medizinischen oder dentalen Instrument 50, 50 A, 50B zu erleichtern, ist die Überwurfmutter 33 entlang Längsachse 39 des Verbindungsadapters 10 und/ oder entlang des zylindrischen Rohrs 40 verschiebbar. Vorzugsweise ist die axiale Länge des Rohrs 40 derart bemessen, dass die Überwurfmutter 33 zwischen einer (in Richtung des Kupplungselements 12) zurückgeschobenen Position, in der insbesondere das freien Ende des Rohrs 40 mit der zumindest einen Öffnung 17 nicht oder nur geringfügig im Innenraum der Überwurfmutter 33 versenkt ist (siehe Figur 2A), und einer vorgeschobenen (von dem Kupplungselement 12 weiter entfernten) Position bewegbar ist, in der insbesondere das freien Ende des Rohrs 40 mit der zumindest einen Öffnung 17 tiefer im Innenraum der Überwurfmutter 33 versenkt ist, so dass die Schraubverbindung der beiden Gewinde 38, 54 möglich ist (siehe Figur 2C).

Das Kupplungselement 12 des Verbindungsadapters 10 umfasst einen zylindrischen Kupplungsfortsatz 13. Der Kupplungsfortsatz 13 erstreckt sich entlang der Längsachse 39 des Verbindungsadapters 10. Am Kupplungselement 12 oder Kupplungsfortsatz 13 ist zumindest eine Dichtung vorgesehen, zum Beispiel ein in einer Nut aufgenommener O-Ring.

Der Verbindungsadapter 10 umfasst zumindest eine, gemäß den Figuren 2A-2C zwei, Medienleitung 11, 15 zur Leitung zumindest eines Aufbereitungs- und/ oder Pflegemediums von der Aufbereitungs- und/ oder Pflegevorrichtung 1,2 14 zu und/ oder in das Instrument 50, 50A, 50B. Die zumindest eine Medienleitung 11, 15 erstreckt sich durch den Verbindungsadapter 10 und verbindet eine erste Öffnung an einer Oberfläche des Verbindungsadapters 10, zum Beispiel an dem freien Ende des zylindrischen Rohrs 40, mit einer zweiten Öffnung an einer Oberfläche des Verbindungsadapters 10, zum Beispiel an dem Kupplungselement 12 oder dem Kupplungsfortsatz 13. Durch die Kopplung des medizinischen oder dentalen Instruments 50, 50A, 50B mit der Aufbereitungs- und/ oder Pflegevorrichtung 1, 2, 14 über den Verbindungsadapters 10 entsteht somit eine Fluidverbindung zur Leitung des Aufbereitungs- und/ oder Pflegemediums von der Aufbereitungs- und/ oder Pflegevorrichtung 1, 2, 14 zu dem aufzubereitenden und/ oder zu pflegenden Instrument 50, 50A, 50B.

Insbesondere in der Figur 2B ist die an dem Kupplungselement 12 oder Kupplungsfortsatz 13 angeordnete Kontaktfläche 32 zu erkennen, die beim Einstecken des Kupplungsfortsatzes 13 in das Kupplungsstück 4 oder die Kupplungsaufnahme 5 der Aufbereitungs- und/ oder Pflegevorrichtung 1, 2, 14 durch Kontakt mit einem Verriegelungsstift 22 des Kupplungsstücks 4 (siehe Figur 6) eine Verriegelung zwischen dem Kupplungsfortsatz 13 und der Kupplungsaufnahme 5 durch das Einschieben des Kupplungsfortsatzes 13 in die Kupplungsaufnahme 5 bewirkt. Die Kontaktfläche 32 umfasst zum Beispiel eine Ringfläche, Ringschulter und/ oder eine Stufe an dem Kupplungselement 12.

Die Figur 2B zeigt auch den an dem Kupplungselement 12 oder Kupplungsfortsatz 13 angeordneten Rücksprung 36, in den die Umfangskante 37 (siehe Figur 8) der Verriegelungsplatte 9 eingreift, wenn der Kupplungsfortsatz 13 in die Kupplungsaufnahme 5 eingesteckt ist, so dass das Kupplungsstück 4 der Aufbereitungs- und/ oder Pflegevorrichtung 1, 2, 14 und das Kupplungselement 12 zuverlässig aneinander befestigt sind. Der Rücksprung 36 umfasst zum Beispiel eine Ringnut oder Aussparung. Um das Kupplungsstück 4 und das Kupplungselement 12 voneinander trennen zu können, muss der Eingriff der Umfangskante 37 in den Rücksprung 36 beendet werden.

Die Figuren 3A - 4B zeigen zwei unterschiedliche medizinische oder dentale Instrumente 50A, 50B und deren Verbindung mit dem Verbindungsadapter 10 der Figuren 2A - 2C.

Das Instrument 50A der Figuren 3A, 3B umfasst ein pneumatisch betriebenes Handstück. Das aus dem Stand der Technik bekannte Handstück umfasst unter anderem ein pneumatisch in Drehung versetzbares Laufrad, eine mit dem Laufrad verbundene und durch das Laufrad rotierbare Werkzeughalterung zur lösbaren Halterung eines Behandlungswerkzeugs, zumindest eine Medienleitungen, die sich vom Kupplungsende 51 durch die hohle Außenhülse 52 in Richtung eines, bis zu einem oder in ein Kopfteil(s) 57 des Handstücks erstreckt, und einen sogenannten Fixanschluss am Kupplungsende 51 des Handstücks. Der Fixanschluss umfasst insbesondere einen oder mehrere Fortsätze 55, die sich von dem Kupplungsende 51 erstrecken sowie das Außengewinde 54. Der zumindest eine Fortsatz 55 umfasst zum Beispiel Endabschnitte der durch das Instrument 50A verlaufenden Medienleitungen und/ oder elektrische Stiftkontakte. Mit Hilfe der Fortsätze 55 und des Außengewindes 54 ist das Instrument 50A (nicht drehbar) mit einem Versorgungsschlauch oder mit dem Verbindungsadapter 10 koppelbar (siehe Figur 3B). Durch diese Kopplung wird oder ist die Einheit des Instruments 50, 50A, 50B und des Verbindungsadapter 10 geschaffen, die in dem in den Figuren 1A - 1F beschriebenen und dargestellten Verfahren zur Aufbereitung und/ oder Pflege eines medizinischen oder dentalen Instruments 50, 50A, 50B aufeinanderfolgend mit den mehreren Aufbereitungs- und/ oder Pflegevorrichtungen 1, 2, 14 verbunden wird. Durch diese Kopplung des Instruments 50A und des Verbindungsadapter 10 ist oder wird insbesondere auch die im Vorstehenden schon mehrmals beschriebene fluidübertragende Verbindung zwischen der Aufbereitungs- und/ oder Pflegevorrichtung 1, 2, 14 und dem Instrument 50A ermöglicht.

Das Instrument 50B der Figuren 4A, 4B umfasst ein Handstück 58 und eine mit dem Handstück 58 verbindbare Drehkupplung 56, die beide aus dem Stand der Technik bekannt sind. Das Handstück 58 umfasst unter anderem eine im Kopfteil angeordnete, in Drehung versetzbare Werkzeughalterung zur lösbaren Halterung eines Behandlungswerkzeugs und zumindest eine Medienleitung, die sich durch die hohle Außenhülse 52 in Richtung eines, bis zu einem oder in ein Kopfteil(s) 57 des Handstücks 58 erstreckt. Das Handstück 58 umfasst auch ein in der Außenhülse 52 angeordnetes, schematisch dargestelltes Bauteil 53. Das Handstück 58 ist in bekannter Weise lösbar und drehbar mit der Drehkupplung 56 verbindbar. Die Drehkupplung 56 ist von zumindest einer Medienleitung zur Versorgung des Handstücks 58 mit einem Medium und gegebenenfalls von elektrischen Leitern zur elektrischen Versorgung des Handstücks 58 durchsetzt. Die Drehkupplung 56 ist mit dem Verbindungsadapter 10 über eine Kupplungsseite 59 lösbar verbunden, wobei die Kupplungsseite 59 ähnlich oder gleich wie das Kupplungsende 51 des Instruments 50A der Figur 3A aufgebaut ist. Insbesondere umfasst die Kupplungsseite 59 zumindest einen Fortsatz 55, der von der Kupplungsseite 59 absteht und in einer Öffnung 17 des Verbindungsadapters 19 aufnehmbar ist, sowie ein Außengewinde 54. Der zumindest eine Fortsatz 55 umfasst zum Beispiel Endabschnitte der durch die Drehkupplung 56 verlaufenden Medienleitungen und/ oder elektrische Stiftkontakte der elektrischen Leiter. Mit Hilfe der Fortsätze 55 und des Außengewindes 54 ist die Drehkupplung 56 und das Instrument 50B mit einem Versorgungsschlauch oder mit dem Verbindungsadapter 10 koppelbar (siehe Figur 4B). Durch diese Kopplung wird oder ist die Einheit des Instruments 50, 50A, 50B und des Verbindungsadapter 10 geschaffen, die in dem in den Figuren 1A - 1F beschriebenen und dargestellten Verfahren zur Aufbereitung und/ oder Pflege eines medizinischen oder dentalen Instruments 50, 50A, 50B aufeinanderfolgend mit den mehreren Aufbereitungs- und/ oder Pflegevorrichtungen 1, 2, 14 verbunden wird. Durch diese Kopplung des Instruments 50B und des Verbindungsadapter 10 ist oder wird insbesondere auch die im Vorstehenden schon mehrmals beschriebene fluidübertragende Verbindung zwischen der Aufbereitungs- und/ oder Pflegevorrichtung 1, 2, 14 und dem Instrument 50B und bevorzugt somit die Aufbereitung und/ oder Pflege auch des im Inneren des Instruments 50B angeordneten Bauteils 53 ermöglicht.

In der Figur 5 ist eine Aufbereitungs- und/ oder Pflegevorrichtung 2 in Form eines Pflegegeräts 2A dargestellt, welches insbesondere dazu vorgesehen ist, das Instrument 50 mit einem Druckgas (Druckluft) durchzublasen und mit einem Schmiermittel zu versorgen. Das aus dem Stand der Technik bekannte Pflegegerät 2A umfasst unter anderem ein Gehäuse 60, das eine Kammer 61 bildet, in der ein aufzubereitendes und/ oder zu pflegendes Instrument 50 aufnehmbar ist. Die Kammer 61 ist durch einen bewegbaren Deckel 62 verschließbar. Das Pflegegerät 2A umfasst des Weiteren zumindest einen Anschluss 63 an eine oder mehrere externe Medienquelle, zum Beispiel an eine Druckluftquelle oder eine Quelle für ein Aufbereitungs- und/ oder Pflegemedium, und/ oder an eine externe Energiequelle. Im Inneren des Gehäuse 60 kann sich zum Beispiel ein Behälter oder eine Aufnahme für einen Behälter für ein Aufbereitungs- und/ oder Pflegemedium befinden. Im Inneren des Gehäuses 60 ist des Weiteren eine Fördervorrichtung für zumindest ein Aufbereitungs- und/ oder Pflegemedium vorgesehen, die zum Beispiel eine Pumpe, Leitungen, Ventile und ähnliche Bauteile und Stellelemente aufweist, so dass das zumindest eine Aufbereitungs- und/ oder Pflegemedium an das in der Kammer 61 angeordnete Kupplungsstück 4 förderbar ist. Vorzugsweise umfasst das Pflegegerät 2A eine elektrische Steuervorrichtung oder einen Controller zur Steuerung des Pflegegeräts 2A, insbesondere des Pflegeprozesses, und gegebenenfalls ein mit der Steuervorrichtung verbundenes Anzeige- und/ oder Bedienpanel für einen Anwender.

Wie aus der Figur 5 zu erkennen ist, ist mittels eines Verbindungsadapters 10 ein Instrument 50 an das Kupplungsstück 4, insbesondere fluidübertragend, angeschlossen, so wie dies zum Beispiel auch in der Figur 1D schematisch dargestellt ist. Somit kann ein Aufbereitungs- und/ oder Pflegemedium an oder in das Instrument 50 gefördert werden. Der Aufbau des Verbindungsadapters 10 und die Verbindung mit dem Instrument 50 entsprechen bevorzugt den Ausführungsbeispielen der Figuren 2A - 4B.

Das Kupplungselement 12 oder der Kupplungsfortsatz 13 des Verbindungsadapters 10 und das Kupplungsstück 4 des Pflegegeräts 2A bilden eine Kupplungsvorrichtung 30, die aufgrund ihres Aufbaus besonders einfach zu handhaben und daher in einem Verfahren zur Aufbereitung und/ oder Pflege gemäß den Figuren 1A - 1F besonders vorteilhaft einsetzbar ist. Das Kupplungselement 12, das den an dem Verbindungsadapter 10 vorgesehenen Abschnitt der Kupplungsvorrichtung 30 bildet, wurde im Vorstehenden, insbesondere in Verbindung mit den Figuren 2A - 2C bereits beschrieben, so dass im Folgenden unter Bezugnahme auf die Figuren 6 - 8 vor allem der Aufbau des Kupplungsstücks 4 und das Zusammenwirken des Kupplungselements 12 und des Kupplungsstücks 4 beschrieben sind.

Das Kupplungsstück 4 weist einen, vorzugsweise zylindrischen, Körper 6 auf, in dem die Kupplungsaufnahme 5 geformt ist, zum Beispiel als zylindrische Bohrung. Die Kupplungsaufnahme 5 ragt zumindest ein Stück in den Körper 6, so dass der Kupplungsfortsatz 13 darin aufnehmbar ist.

An dem Körper 6 ist eine beweglich, insbesondere verschiebbar angeordnete Verriegelungsplatte 9, vorgesehen, die wahlweise in eine Verriegelungsposition und eine entriegelte Position bewegbar ist. In der Verriegelungsplatte 9 ist eine Durchgangsöffnung 20 vorgesehen, wobei die Durchgangsöffnung 20 mit der Öffnung einer Medienleitung 7 und/ oder mit der Kupplungsaufnahme 5 zum Durchtritt des Aufbereitungs- und/ oder Pflegemediums fluidverbunden ist. Die Durchgangsöffnung 20 ist derart bemessen, dass zumindest ein Teil des Kupplungsfortsatzes 13 des Kupplungselements 12 darin aufnehmbar ist.

In dem Körper 6 ist eine Nut 64 vorgesehen, in welcher die Verriegelungsplatte 9 mit der Durchgangsöffnung 20 beweglich oder verschiebbar aufgenommen und insbesondere geführt ist. Dazu sind die Ränder der Verriegelungsplatte 9 in der Nut 64, insbesondere in Führungen der Nut 64 aufgenommen.

An der Verriegelungsplatte 9 oder als Teil davon ist ein Stellelement 65 vorgesehen, wodurch das Betätigen der Verriegelungsplatte 9, insbesondere, wenn diese von der Verriegelungsposition in die entriegelte Position bewegt wird, für den Anwender erleichtert wird. Das Stellelement 65 ist plattenförmig geformt und gewinkelt zum dem Abschnitt der Verriegelungsplatte 9, in dem sich die Durchgangsöffnung 20 befindet, angeordnet. Ein erstes Vorspannelement 21, zum Beispiel in Form einer Spiralfeder, spannt die Verriegelungsplatte 9 vor, insbesondere in die Verriegelungsposition. Das erste Vorspannelement 21 ist vorzugsweise zwischen dem Körper 6, zum Beispiel in einer Aufnahme des Körpers 6, und dem Stellelement 65 angeordnet. Das erste Vorspannelement 21 ist insbesondere an einer dem Körper 6 zugewandten Unterseite des Stellelements 65 vorgesehen.

An dem Kupplungsstück 4 ist zumindest eine Medienleitung 7 zur Leitung eines Aufbereitungs- und/ oder Pflegemediums durch den Körper 6 vorgesehen, wobei die Medienleitung 7 sich durch zumindest einen Teil des Körpers 6 erstreckt. Die Medienleitung 7 endet in einer Öffnung 8 an einer Oberfläche der Kupplungsaufnahme 5 und/ oder ist mit der Kupplungsaufnahme 5 und der Durchgangsöffnung 20 fluidleitend verbunden.

In einer im Wesentlichen rechtwinkelig zur Verriegelungsplatte 9 angeordneten Bohrung 23 des Körpers 6 ist einen Verriegelungsstift 22 verschiebbar aufgenommen. Der Verriegelungsstift 22 weist ein Kontaktende 35, ein Verriegelungsstück 25 und ein Entriegelungsstück 26 auf. Das Kontaktende 35 ragt als freies Ende über den Körper 6 hinaus und ist somit durch einen Teil des Kupplungselements 12 oder des Kupplungsfortsatzes 13, insbesondere durch die Kontaktfläche 32, kontaktierbar. Der Verriegelungsstift 22 ist durch ein in der Bohrung 23 angeordnetes zweites Vorspannelement 29, zum Beispiel eine Spiralfeder, vorgespannt, insbesondere in die entriegelte Position, in welcher das Kontaktende 35 weiter vom Körper 6 entfernt ist als in der Verriegelungsposition. Der Außendurchmesser des Verriegelungsstücks 25 ist geringer ist als der Außendurchmesser des Entriegelungsstücks 26.

In der Verriegelungsplatte 9 ist eine Verriegelungsöffnung 24 vorgesehen, durch welche sich der Verriegelungsstift 22 erstreckt. Die Verriegelungsöffnung 24 weist eine längliche Schlitzform auf und öffnet sich in die Durchgangsöffnung 20. Die Verriegelungsöffnung 24 umfasst einen Verriegelungsabschnitt 27 zur Aufnahme des Verriegelungsstücks 25 des Verriegelungsstifts 22, und einen Entriegelungsabschnitt 28 zur Aufnahme des Entriegelungsstücks 26 des Verriegelungsstifts 22. Der Innendurchmesser des Verriegelungsabschnitts 27 ist, insbesondere in der Ebene, in der sich die Verriegelungsplatte 9 erstreckt, geringer als der Innendurchmesser des Entriegelungsabschnitt 28. Der Entriegelungsabschnitt 28 ist näher zur Durchgangsöffnung 20 angeordnet als der Verriegelungsabschnitt 27.

Die Verriegelungsplatte 9 und die Durchgangsöffnung 20 sind wahlweise in die Verriegelungsposition und die entriegelte Position bewegbar und darin mit Hilfe des Verriegelungsstifts 22 und der Verriegelungsöffnung 24 fixierbar. In der Verriegelungsposition sind die Kupplungsaufnahme 5 und der Kupplungsfortsatz 13 und/ oder das Kupplungsstück 4 und das Kupplungselement 12, insbesondere fluidübertragend, miteinander verbunden, wohingegen in der entriegelten Position die genannten Elemente 4, 12; 5, 13, voneinander getrennt oder trennbar sind.

Der Wechsel zwischen der Verriegelungsposition und der in den Figuren 7 und 8 dargestellten entriegelten Position ist durch wahlweise Krafteinwirkung auf die Verriegelungsplatte 9 und den Verriegelungsstift 22 und die dadurch verursachte Bewegung der Verriegelungsplatte 9 und des Verriegelungsstifts 22 bewirkt. Wird zum Beispiel der Verriegelungsstift 22 beim Einstecken des Kupplungselements 4 in die Kupplungsaufnahme 5, insbesondere durch die Kontaktfläche 32, gegen die Kraft des zweiten Vorspannelements 29 ein Stück in die Bohrung 23 bewegt, so wird (anstelle des sich in der Verriegelungsöffnung 24 befindlichen Entriegelungsstücks 26) das Verriegelungsstück 25 des Verriegelungsstifts 22 in die Verriegelungsöffnung 24 verschoben, wobei aufgrund des geringeren Durchmessers des Verriegelungsstücks 25 und durch die Vorspannkraft des ersten Vorspannelements 21 die Verriegelungsplatte 9 (aus der entriegelten Position) in die Verriegelungsposition bewegt und das Verriegelungsstück 25 in dem Verriegelungsabschnitt 27 der Verriegelungsöffnung 24 aufgenommen werden.

Die Durchgangsöffnung 20 der Verriegelungsplatte 9 ist durch eine Umfangskante 37 begrenzt, die aufgrund der Bewegung der Verriegelungsplatte 9 in die Verriegelungsposition in den Rücksprung 36 des in das Kupplungselement 4 eingesteckten Kupplungsfortsatzes 13 eingreift, wodurch das Kupplungsstück 4 und das Kupplungselement 12 aneinander befestigt sind. Die Verriegelungsplatte 9 und die Durchgangsöffnung 20 nehmen somit insbesondere in der Verriegelungsposition eine Position ein, in welcher die Umfangskante 37 in oder über die Kupplungsaufnahme 5 ragt, vorzugsweise sind die Durchgangsöffnung 20 und die Kupplungsaufnahme 5 zueinander verschoben oder exzentrisch angeordnet, wodurch die Umfangskante 37 in den Rücksprung 36 eingreift. Hingegen nehmen die Verriegelungsplatte 9 und die Durchgangsöffnung 20 insbesondere in der entriegelten Position eine Position ein, in welcher die Umfangskante 37 nicht in oder über die Kupplungsaufnahme 5 ragt, vorzugsweise sind die Durchgangsöffnung 20 und die Kupplungsaufnahme 5 zumindest annähernd konzentrisch angeordnet, wodurch das Kupplungsstück 4 und das Kupplungselement 12 voneinander lösbar sind. Diese beschriebene Verschiebung der Umfangskante 37 bewirkt die Kupplung und Lösbarkeit der Kupplungsvorrichtung 30 oder des Kupplungsstücks 4 und des Kupplungselements 12 durch Eingriff oder Nicht-Eingriff der Umfangskante 37 in den Rücksprung 36.

Durch eine Krafteinwirkung an der Verriegelungsplatte 9, insbesondere deren Stellelement 65, entgegen der Vorspannung des ersten Vorspannelements 21 bewegt oder verschiebt sich die Verriegelungsplatte 9 relativ zu dem Verriegelungsstift 22, so dass dieser von dem Entriegelungsabschnitt 28 der Verriegelungsöffnung 24 aufgenommen wird, wobei aufgrund der Vorspannkraft des zweiten Vorspannelements 29 und aufgrund des größeren Durchmessers des Entriegelungsabschnitts 28 der Verriegelungsstift 22 sich (selbsttätig) teilweise aus der Bohrung 23 des Körpers 6 wegbewegt bis dessen Entriegelungsstück 26 in dem Entriegelungsabschnitt 28 aufgenommen ist, wodurch die Verriegelungsplatte 9 die entriegelte Position einnimmt und in dieser fixiert ist.

Die beschriebenen oder dargestellten Ausführungsbeispiele dienen der Veranschaulichung der Erfindung. Die in einem Ausführungsbeispiel offenbarten Merkmale sind daher nicht auf dieses Ausführungsbeispiel beschränkt, sondern sind einzeln oder gemeinsam mit einem oder mehreren Merkmalen eines der anderen Ausführungsbeispiele kombinierbar.

## Patentansprüche

1. Verfahren zur Aufbereitung und/ oder Pflege eines medizinischen oder dentalen Instruments (50, 50A, 50B), **gekennzeichnet durch** die Schritte:
a) Bereitstellen eines Verbindungsadapters (10), der lösbar mit einem Kupplungsende (51) des medizinischen oder dentalen Instruments (50, 50A, 50B) verbindbar ist;
b) Verbinden des medizinischen oder dentalen Instruments (50, 50A, 50B) über den Verbindungsadapter (10) mit einer ersten Aufbereitungs- und/ oder Pflegevorrichtung (1) für ein medizinisches oder dentales Instrument (50, 50A, 50B);
c) Aufbereitung und/ oder Pflege des medizinischen oder dentalen Instruments (50, 50A, 50B) durch die erste Aufbereitungs- und/ oder Pflegevorrichtung (1);
d) Trennen des Verbindungsadapters (10) gemeinsam mit dem daran befestigten medizinischen oder dentalen Instrument (50, 50A, 50B) von der ersten Aufbereitungs- und/ oder Pflegevorrichtung (1);
e) Verbinden des Verbindungsadapters (10) gemeinsam mit dem daran befestigten medizinischen oder dentalen Instrument (50, 50A, 50B) mit einer zweiten Aufbereitungs- und/ oder Pflegevorrichtung (2, 14) für ein medizinisches oder dentales Instrument (50, 50A, 50B), wobei die erste und die zweite Aufbereitungs- und/ oder Pflegevorrichtung (1; 2, 14) unterschiedliche Aufbereitungs- und/ oder Pflegevorrichtung sind, die unterschiedliche Aufbereitungs- und/ oder Pflegebehandlungen an dem medizinischen oder dentalen Instrument (50, 50A, 50B) anwenden;
f) Aufbereitung und/ oder Pflege des medizinischen oder dentalen Instruments (50, 50A, 50B) durch die zweite Aufbereitungs- und/ oder Pflegevorrichtung (2, 14).

2. Verfahren zur Aufbereitung und/ oder Pflege nach Anspruch 1, **dadurch gekennzeichnet, dass** der Schritt b) folgenden Teilschritte umfasst:
b1) Verbinden des medizinischen oder dentalen Instruments (50, 50A, 50B) mit dem Verbindungsadapter (10);
b2) Verbinden des Verbindungsadapters (10) gemeinsam mit dem daran befestigten medizinischen oder dentalen Instrument (50, 50A, 50B) mit der ersten Aufbereitungs- und/ oder Pflegevorrichtung (1).

3. Verfahren zur Aufbereitung und/ oder Pflege nach Anspruch 1 oder 2, **dadurch gekennzeichnet, dass** nach dem Schritt f) das medizinische oder dentale Instrument (50, 50A, 50B) von dem Verbindungsadapter (10) getrennt wird.

4. Verfahren zur Aufbereitung und/ oder Pflege nach einem der vorstehenden Ansprüche, **dadurch gekennzeichnet, dass** das medizinische oder dentale Instrument (50, 50A, 50B) eine hohle Außenhülse (52) aufweist, in deren Inneren zumindest ein Bauteil (53) angeordnet ist, wobei die Aufbereitung und/ oder Pflege des medizinischen oder dentalen Instruments (50, 50A, 50B) durch die erste Aufbereitungs- und/ oder Pflegevorrichtung (1) und/ oder durch die zweite Aufbereitungs- und/ oder Pflegevorrichtung (2, 14) eine Aufbereitung und/ oder Pflege des zumindest einen im Inneren der hohlen Außenhülse (52) angeordneten Bauteils (53) umfasst, insbesondere durch Leiten eines Aufbereitungs- und/ oder Pflegemediums in Richtung oder zu dem zumindest einen Bauteil (53).

5. Verfahren zur Aufbereitung und/ oder Pflege nach einem der vorstehenden Ansprüche, **dadurch gekennzeichnet, dass**
der Verbindungsadapter (10) und die erste Aufbereitungs- und/ oder Pflegevorrichtung (1) und/ oder der Verbindungsadapter (10) und die zweite Aufbereitungs- und/ oder Pflegevorrichtung (2, 14) durch eine Steckverbindung (3) miteinander verbunden werden.

6. Verfahren zur Aufbereitung und/ oder Pflege nach einem der vorstehenden Ansprüche, **dadurch gekennzeichnet, dass**
die erste Aufbereitungs- und/ oder Pflegevorrichtung (1) eine der folgenden Vorrichtungen umfasst: ein Pflegegerät zur Abgabe eines Pflegemediums an das medizinische oder dentale Instrument (50, 50A, 50B); ein Aufbereitungs- oder Reinigungsgerät zur Abgabe eines Aufbereitungs- oder Reinigungsmediums an das medizinische oder dentale Instrument (50, 50A, 50B); ein Desinfektionsgerät zur Abgabe eines Desinfektionsmediums an das medizinische oder dentale Instrument (50, 50A, 50B); einen Sterilisator oder Autoklaven zur Abgabe eines Sterilisationsmediums an das medizinische oder dentale Instrument (50, 50A, 50B); und dass die zweite Aufbereitungs- und/ oder Pflegevorrichtung (2, 14) eine der folgenden Vorrichtungen, jedoch nicht eine mit der ersten Aufbereitungs- und/ oder Pflegevorrichtung (1) gleiche Vorrichtung umfasst: ein Pflegegerät zur Abgabe eines Pflegemediums an das medizinische oder dentale Instrument (50, 50A, 50B); ein Aufbereitungs- oder Reinigungsgerät zur Abgabe eines Aufbereitungs- oder Reinigungsmediums an das medizinische oder dentale Instrument (50, 50A, 50B); ein Desinfektionsgerät zur Abgabe eines Desinfektionsmediums an das medizinische oder dentale Instrument (50, 50A, 50B); einen Sterilisator oder Autoklaven zur Abgabe eines Sterilisationsmediums an das medizinische oder dentale Instrument (50, 50A, 50B).

7. Verfahren zur Aufbereitung und/ oder Pflege nach einem der vorstehenden Ansprüche, **dadurch gekennzeichnet, dass**
die unterschiedlichen Aufbereitungs- und/ oder Pflegebehandlungen der ersten und der zweiten Aufbereitungs- und/ oder Pflegevorrichtung (1; 2, 14) umfassen, dass das medizinische oder dentale Instrument (50, 50A, 50B) mit zumindest einem unterschiedlichen Aufbereitungs- und/ oder Pflegemedium versorgt wird.

8. System (100) zur Aufbereitung und/ oder Pflege eines medizinischen oder dentalen Instruments (50, 50A, 50B) in einem Verfahren gemäß einem der vorstehenden Ansprüche, mit: einer ersten Aufbereitungs- und/ oder Pflegevorrichtung (1) für ein medizinisches oder dentales Instrument, einer zweiten Aufbereitungs- und/ oder Pflegevorrichtung (2, 14) für ein medizinisches oder dentales Instrument und einem Verbindungsadapter (10), der lösbar mit einem Kupplungsende (51) eines medizinischen oder dentalen Instruments (50, 50A, 50B) und mit der ersten Aufbereitungs- und/ oder Pflegevorrichtung (1) für ein medizinisches oder dentales Instrument und der zweiten Aufbereitungs- und/ oder Pflegevorrichtung (2, 14) für ein medizinisches oder dentales Instrument verbindbar ist, **dadurch gekennzeichnet, dass** die erste Aufbereitungs- und/ oder Pflegevorrichtung (1) und die zweite Aufbereitungs- und/ oder Pflegevorrichtung (2, 14) unterschiedliche Aufbereitungs- und/ oder Pflegevorrichtung sind und unterschiedliche Aufbereitungs- und/ oder Pflegeprozesse an dem medizinischen oder dentalen Instrument (50, 50A, 50B) anwenden, derart, dass nur die erste Aufbereitungs- und/ oder Pflegevorrichtung (1) oder die zweite Aufbereitungs- und/ oder Pflegevorrichtung (2, 14) ausgebildet ist, ein Schmiermittel an das medizinische oder dentale Instrument (50, 50A, 50B) abzugeben.

9. System (100) zur Aufbereitung und/ oder Pflege eines medizinischen oder dentalen Instruments (50, 50A, 50B) nach Anspruch 8, **dadurch gekennzeichnet, dass** die erste Aufbereitungs- und/ oder Pflegevorrichtung (1) und die zweite Aufbereitungs- und/ oder Pflegevorrichtung (2, 14) jeweils zumindest ein Kupplungsstück (4) aufweisen, an welches der Verbindungsadapter (10) durch eine Steckverbindung (3) anschließbar ist.

10. System (100) zur Aufbereitung und/ oder Pflege eines medizinischen oder dentalen Instruments (50, 50A, 50B) nach Anspruch 8 oder 9, **dadurch gekennzeichnet, dass** nur eine der Aufbereitungs- und/ oder Pflegevorrichtungen (1, 2, 14) ausgebildet ist, ein Schmiermittel an das medizinische oder dentale Instrument (50, 50A, 50B) abzugeben, und die andere Aufbereitungs- und/ oder Pflegevorrichtung (1, 2, 14) als Sterilisator oder Autoklav zur Abgabe eines Sterilisationsmediums an das medizinische oder dentale Instrument (50, 50A, 50B) ausgebildet ist.

11. System (100) zur Aufbereitung und/ oder Pflege eines medizinischen oder dentalen Instruments (50, 50A, 50B) nach Anspruch 8, 9 oder 10, **gekennzeichnet durch** eine Kupplungsvorrichtung (30) zur Verbindung der ersten und zweiten Aufbereitungs- und/ oder Pflegevorrichtung (1, 2, 14) zur Aufbereitung und/ oder Pflege eines medizinischen oder dentalen Instruments (50, 50A, 50B) mit dem Verbindungsadapter (10), um ein aufzubereitendes und/ oder zu pflegendes medizinisches oder dentales Instrument (50, 50A, 50B) mit der ersten und zweiten Aufbereitungs- und/ oder Pflegevorrichtung (1, 2, 14) fluidübertragend zu verbinden, wobei die Kupplungsvorrichtung (30) als Steckverbindung (3) ausgebildet ist und ein an der ersten und zweiten Aufbereitungs- und/ oder Pflegevorrichtung (1, 2, 14) vorgesehenes Kupplungsstück (4) sowie ein an dem Verbindungsadapter (10) vorgesehenes Kupplungselement (12) aufweist, **dadurch gekennzeichnet, dass** eines der beiden Bauteile, nämlich das Kupplungsstück (4) oder das Kupplungselement (12), einen Kupplungsfortsatz (13) und das andere der beiden Bauteile, nämlich das Kupplungsstück (4) oder das Kupplungselement (12), eine Kupplungsaufnahme (5) zur Steckaufnahme des Kupplungsfortsatzes (13) aufweist, wobei jenes der beiden Bauteile, nämlich das Kupplungsstück (4) oder das Kupplungselement (12), mit der Kupplungsaufnahme (5) umfasst: einen Körper (6), in dem die Kupplungsaufnahme (5) geformt ist, zumindest eine Medienleitung (7) zur Leitung eines Aufbereitungs- und/ oder Pflegemediums durch den Körper (6), wobei die Medienleitung (7) in einer Öffnung (8) an einer Oberfläche der Kupplungsaufnahme (5) endet, eine in dem Körper (6) beweglich angeordnete Verriegelungsplatte (9), die wahlweise in eine Verriegelungsposition und eine entriegelte Position bewegbar ist, wobei in der Verriegelungsplatte (9) eine Durchgangsöffnung (20) angeordnet ist, wobei die Durchgangsöffnung (20) mit der Öffnung (8) der Medienleitung (7) zum Durchtritt des Aufbereitungs- und/ oder Pflegemediums fluidverbunden ist, ein erstes Vorspannelement (21) zum Vorspannen der Verriegelungsplatte (9), einen Verriegelungsstift (22), der verschiebbar in einer Bohrung (23) des Körpers (6) aufgenommen ist, wobei sich der Verriegelungsstift (22) durch eine Verriegelungsöffnung (24) in der Verriegelungsplatte (9) erstreckt und ein Verriegelungsstück (25) und ein Entriegelungsstück (26) aufweist, wobei die Verriegelungsöffnung (24) einen Verriegelungsabschnitt (27) und einen Entriegelungsabschnitt (28) aufweist, und ein zweites Vorspannelement (29) zum Vorspannen des Verriegelungsstifts (22) derart, dass ein Kontaktende (35) des Verriegelungsstifts (22) über die Verriegelungsplatte (9) hinausragt, wobei die Verriegelungsplatte (9) und der Verriegelungsstift (22) derart zusammenwirken, dass durch eine Krafteinwirkung an dem Kontaktende (35) des Verriegelungsstifts (22) das Kontaktende (35) entgegen der Vorspannung des zweiten Vorspannelements (29) in Richtung der Bohrung (23) des Körpers (6) bewegbar ist, bis das Verriegelungsstück (25) des Verriegelungsstifts (22) in dem Verriegelungsabschnitt (27) der Verriegelungsöffnung (24) aufgenommen ist, wodurch die Verriegelungsplatte (9) aufgrund der Vorspannkraft des ersten Vorspannelements (21) in die Verriegelungsposition bewegbar ist, und wobei die Verriegelungsplatte (9) und der Verriegelungsstift (22) derart zusammenwirken, dass durch eine Krafteinwirkung an der Verriegelungsplatte (9) entgegen der Vorspannung des ersten Vorspannelements (21) die Verriegelungsplatte (9) in die entriegelt Position bewegt wird, wodurch aufgrund der Vorspannkraft des zweiten Vorspannelements (29) das Kontaktende (35) des Verriegelungsstifts (22) von der Bohrung (23) des Körpers (6) wegbewegbar ist, wodurch das Entriegelungsstück (26) des Verriegelungsstifts (22) in dem Entriegelungsabschnitt (26) der Verriegelungsöffnung (24) aufgenommen wird und die Verriegelungsplatte (9) in der entriegelten Position fixiert ist.

12. System (100) zur Aufbereitung und/ oder Pflege eines medizinischen oder dentalen Instruments (50, 50A, 50B) nach Anspruch 11, **dadurch gekennzeichnet, dass** der Außendurchmesser des Verriegelungsstücks (25) des Verriegelungsstifts (22) geringer ist als der Außendurchmesser des Entriegelungsstücks (26) des Verriegelungsstifts (22) und dass der Innendurchmesser des Verriegelungsabschnitts (27) der Verriegelungsöffnung (24) geringer ist als der Innendurchmesser des Entriegelungsabschnitts (28) der Verriegelungsöffnung (24).

13. System (100) zur Aufbereitung und/ oder Pflege eines medizinischen oder dentalen Instruments (50, 50A, 50B) nach einem der Ansprüche 11 oder 12, **dadurch gekennzeichnet, dass**
an dem andern Bauteil, nämlich dem Kupplungsstück (4) oder dem Kupplungselement (12), mit dem Kupplungsfortsatz (13) ein Rücksprung (36) vorgesehen ist, in den eine Umfangskante (37) der Durchgangsöffnung (20) der Verriegelungsplatte (9) eingreift, wenn der Kupplungsfortsatz (13) in die Kupplungsaufnahme (5) eingesteckt ist und die Verriegelungsplatte (9) die Verriegelungsposition einnimmt, so dass das Kupplungsstück (4) und das Kupplungselement (12) aneinander befestigt sind.

14. System (100) zur Aufbereitung und/ oder Pflege eines medizinischen oder dentalen Instruments (50, 50A, 50B) nach einem der Ansprüche 11 - 13, **dadurch gekennzeichnet, dass** an dem Verbindungsadapter (10) ein Anschlusselement (34) zum Anschluss eines aufzubereitenden und/ oder zu pflegenden medizinischen oder dentalen Instruments (50, 50A, 50B) vorgesehen ist, wobei das Anschlusselement (34) eine Überwurfmutter (33) mit Innengewinde (38) zur Schraubverbindung mit einem Gewinde (54) des medizinischen oder dentalen Instruments (50, 50A, 50B) aufweist, wobei die Überwurfmutter (33) entlang einer Längsachse (39) des Anschlusselements (34) verschiebbar ist.

15. System (100) zur Aufbereitung und/ oder Pflege eines medizinischen oder dentalen Instruments (50, 50A, 50B) nach einem der Ansprüche 11 - 14, **dadurch gekennzeichnet, dass** an dem anderen Bauteile, nämlich dem Kupplungsstück (4) oder dem Kupplungselement (12), mit dem Kupplungsfortsatz (13) eine Kontaktfläche (32) vorgesehen ist, die derart angeordnet ist, dass sie beim Einstecken des Kupplungsfortsatzes (13) in die Kupplungsaufnahme (5) die Krafteinwirkung auf das Kontaktende (35) des Verriegelungsstifts (22) bewirkt, um das Kontaktende (35) entgegen der Vorspannung des zweiten Vorspannelements (29) in Richtung der Bohrung (23) des Körpers (6) zu bewegen, bis das Verriegelungsstück (25) des Verriegelungsstifts (9) in dem Verriegelungsabschnitt (27) der Verriegelungsöffnung (24) aufgenommen ist, wodurch die Verriegelungsplatte (9) aufgrund der Vorspannkraft des ersten Vorspannelements (21) in die Verriegelungsposition bewegt wird.

## Claims

1. A method for preparing and/or caring for a medical or dental instrument (50, 50A, 50B), **characterised by** the steps of:
a) providing a connection adapter (10) which is releasably connectable to a coupling end (51) of the medical or dental instrument (50, 50A, 50B);
b) connecting the medical or dental instrument (50, 50A, 50B) via the connection adapter (10) to a first preparation and/or care device (1) for a medical or dental instrument (50, 50A, 50B);
c) preparing and/or caring for the medical or dental instrument (50, 50A, 50B) by the first preparation and/or care device (1);
d) separating the connection adapter (10), jointly with the medical or dental instrument (50, 50A, 50B) attached thereto, from the first preparation and/or care device (1);
e) connecting the connection adapter (10), jointly with the medical or dental instrument (50, 50A, 50B) attached thereto, to a second preparation and/or care device (2, 14) for a medical or dental instrument (50, 50A, 50B), wherein the first and second preparation and/or care device (1, 2, 14) are different preparation and/or care devices which apply different preparation and/or care processes to the medical or dental instrument;
f) preparing and/or caring for the medical or dental instrument (50, 50A, 50B) by the second preparation and/or care device (2, 14).

2. The method for preparation and/or care according to claim 1, **characterised in that** step b) comprises the following sub-steps:
b1) connecting the medical or dental instrument (50, 50A, 50B) to the connection adapter (10);
b2) connecting the connection adapter (10), jointly with the medical or dental instrument (50, 50A, 50B) attached thereto, to the first preparation and/or care device (1).

3. The method for preparation and/or care according to claim 1 or 2, **characterised in that** after step f), the medical or dental instrument (50, 50A, 50B) is separated from the connection adapter (10).

4. The method for preparation and/or care according to one of the preceding claims, **characterised in that**
the medical or dental instrument (50, 50A, 50B) comprises a hollow outer sleeve (52), in which at least one component (53) is arranged, wherein the preparation and/or care of the medical or dental instrument (50, 50A, 50B) by the first preparation and/or care device (1) and/or by the second preparation and/or care device (2, 14) comprises preparation and/or care of the at least one component (53) arranged inside the hollow outer sleeve (52), in particular by directing a preparation and/or care medium towards or to the at least one component (53).

5. The method for preparation and/or care according to one of the preceding claims, **characterised in that**
the connection adapter (10) and the first preparation and/or care device (1) and/or the connection adapter (10) and the second preparation and/or care device (2, 14) are connected to each other by a plug-in connector (3).

6. The method for preparation and/or care according to one of the preceding claims, **characterised in that**
the first preparation and/or care device (1) comprises one of the following devices: a care device for delivering a care medium to the medical or dental instrument (50, 50A, 50B); a preparation or cleaning device for delivering a preparation or cleaning medium to the medical or dental instrument (50, 50A, 50B); a disinfection device for delivering a disinfection medium to the medical or dental instrument (50, 50A, 50B); a steriliser or autoclave for delivering a sterilisation medium to the medical or dental instrument (50, 50A, 50B) and **in that** the second preparation and/or care device (2, 14) comprises one of the following devices, but not a device equal to the first preparation and/or care device (1): a care device for delivering a care medium to the medical or dental instrument (50, 50A, 50B); a preparation or cleaning device for delivering a preparation or cleaning medium to the medical or dental instrument (50, 50A, 50B); a disinfection device for delivering a disinfection medium to the medical or dental instrument (50, 50A, 50B); a steriliser or autoclave for delivering a sterilisation medium to the medical or dental instrument (50, 50A, 50B).

7. The method for preparation and/or care according to one of the preceding claims, **characterised in that**
the different preparation and/or care processes of the first and second preparation and/or care device (1; 2, 14) comprise that the medical or dental instrument (50, 50A, 50B) is supplied with at least one different preparation and/or care medium.

8. A system (100) for preparing and/or caring for a medical or dental instrument (50, 50A, 50B) in a method according to one of the preceding claims, having: a first preparation and/or care device (1) for a medical or dental instrument, a second preparation and/or care device (2, 14) for a medical or dental instrument, and a connection adapter (10) detachably connectable to a coupling end (51) of a medical or dental instrument (50, 50A, 50B) and to the first preparation and/or maintenance device (1) for a medical or dental instrument and to the second preparation and/or maintenance device (2, 14) for a medical or dental instrument, characterized int that
the first preparation and/or care device (1) and the second preparation and/or care device (2, 14) are different preparation and/or care devices and apply different preparation and/or care processes to the medical or dental instrument (50, 50A, 50B), such, that only the the first preparation and/or maintenance device (1) or the second preparation and/or maintenance device (2, 14) is configured to deliver a lubricant to the medical or dental instrument (50, 50A, 50B).

9. The system (100) for preparing and/or caring for a medical or dental instrument (50, 50A, 50B) according to claim 8, **characterised in that**
the first preparation and/or care device (1) and the second preparation and/or care device (2, 14) each have at least one coupling piece (4) to which the connection adapter (10) can be connected by a plug-in connection (3).

10. The system (100) for preparing and/or caring for a medical or dental instrument (50, 50A, 50B) according to claim 8 or 9, **characterised in that**
only one of the preparation and/or care devices (1, 2, 14) is configured to deliver a lubricant to the medical or dental instrument (50, 50A, 50B) and the other preparation and/or care device (1, 2, 14) is configured as steriliser or autoclave for delivering a sterilisation medium to the medical or dental instrument (50, 50A, 50B).

11. A system (100) for preparing and/or caring for a medical or dental instrument (50, 50A, 50B) according to claim 8, 9 or 10, **characterised by**
a coupling device (30) for connecting the first and second preparation and/or care device (1, 2, 14) for preparing and/or caring for a medical or dental instrument (50, 50A, 50B) to the connection adapter (10) to connect a medical or dental instrument (50, 50A, 50B) to be prepared and/or cared for to the first and second preparation and/or care device (1, 2, 14) in a fluid-transferring manner, the coupling device (30) being configured as a plug-in connection (3) and having a coupling piece (4) provided on the first and second preparation and/or care device (1, 2, 14) and a coupling element (12) provided on the connection adapter (10), **characterised in that**
one of the two components, namely the coupling piece (4) or the coupling element (12), comprises a coupling extension (13) and the other of the two components, namely the coupling piece (4) or the coupling element (12), comprises a coupling receptacle (5) for plug-in reception of the coupling extension (13), wherein that one of the two components, namely the coupling piece (4) or the coupling element (12), with the coupling receptacle (5) comprises: a body (6), in which the coupling receptacle (5) is formed, at least one media line (7) for conducting a preparation and/or care medium through the body (6), the media line (7) terminating in an opening (8) on a surface of the coupling receptacle (5), a locking plate (9) movably arranged in the body (6), which is selectively movable into a locking position and an unlocked position, a through-opening (20) being arranged in the locking plate (9), the through-opening (20) being fluidically connected to the opening (8) of the media line (7) for the passage of the preparation and/or care medium, a first pretensioning element (21) for pretensioning the locking plate (9), a locking pin (22) which is slidably received in a bore (23) of the body (6), the locking pin (22) extending through a locking opening (24) in the locking plate (9) and comprising a locking piece (25) and an unlocking piece (26), the locking opening (24) having a locking portion (27) and an unlocking portion (28), and a second pretensioning element (29) for pretensioning the locking pin (22) such that a contact end (35) of the locking pin (22) projects beyond the locking plate (9), wherein the locking plate (9) and the locking pin (22) interact in such a way that, as a result of a force acting on the contact end (35) of the locking pin (22), the contact end (35) can be moved in the direction of the bore (23) of the body (6) counter to the pretensioning of the second pretensioning element (29), until the locking piece (25) of the locking pin (22) is received in the locking portion (27) of the locking opening (24), so that the locking plate (9) is movable into the locking position due to the pretensioning force of the first pretensioning element (21), and wherein the locking plate (9) and the locking pin (22) interact in such a way that the locking plate (9) is moved into the unlocked position by a force acting on the locking plate (9) against the pretensioning force of the first pretensioning element (21), so that, due to the pretensioning force of the second pretensioning element (29), the contact end (35) of the locking pin (22) can be moved away from the bore (23) of the body (6), so that the unlocking piece (26) of the locking pin (22) is received in the unlocking section (26) of the locking opening (24) and the locking plate (9) is fixed in the unlocked position.

12. The system (100) for preparing and/or caring for a medical or dental instrument (50, 50A, 50B) according to claim 11, **characterised in that**
the outer diameter of the locking piece (25) of the locking pin (22) is smaller than the outer diameter of the unlocking piece (26) of the locking pin (22), and **in that** the inner diameter of the locking section (27) of the locking opening (24) is smaller than the inner diameter of the unlocking section (28) of the locking opening (24).

13. The system (100) for preparing and/or caring for a medical or dental instrument (50, 50A, 50B) according to claim 11 or 12, **characterised in that**
a recess (36) is provided on the other component, namely the coupling piece (4) or the coupling element (12), having the coupling extension (13), in which recess (36) a circumferential edge (37) of the through-opening (20) of the locking plate (9) engages when the coupling extension (13) is inserted into the coupling receptacle (5) and the locking plate (9) assumes the locking position, so that the coupling piece (4) and the coupling element (12) are secured to one another.

14. The system (100) for preparing and/or caring for a medical or dental instrument (50, 50A, 50B) according to one of claims 11 - 13, **characterised in that**
a connection element (34) for connecting a medical or dental instrument (50, 50A, 50B) to be prepared and/or cared for is provided on the connection adapter (10), the connection element (34) having a union nut (33) with an internal thread (38) for screwed connection with a thread (54) of the medical or dental instrument (50, 50A, 50B), the union nut (33) being displaceable along a longitudinal axis (39) of the connection element (34).

15. The system (100) for preparing and/or caring for a medical or dental instrument (50, 50A, 50B) according to one of claims 11 - 14, **characterised in that**
a contact surface (32) is provided on the other component, namely the coupling piece (4) or the coupling element (12), having the coupling extension (13), which contact surface (32) is arranged in such a way that, when the coupling extension (13) is inserted into the coupling receptacle (5), it exerts the force on the contact end (35) of the locking pin (22), in order to move the contact end (35) in the direction of the bore (23) of the body (6) against the pretension of the second pretensioning element (29) until the locking piece (25) of the locking pin (9) is received in the locking portion (27) of the locking opening (24), such that the locking plate (9) is moved into the locking position due to the pretensioning force of the first pretensioning element (21).

## Revendications

1. Procédé de préparation **et/ou d'entretien d'un instrument médical ou dentaire** (50, 50A, 50B), **caractérisé par** les étapes suivantes:
a) **la fourniture d'un adaptateur de connexion (10) qui peut être raccordé de manière** détachable à une extrémité **de couplage (51) de l'instrument médical ou dentaire (50,** 50A, 50B);
b) **le raccordement de l'instrument médical ou dentaire** (50, 50A, 50B) **via l'adaptateur de** connexion (10) à un premier dispositif de préparation et/ou d'entretien (1) pour un instrument médical ou dentaire (50, 50A, 50B);
c) la préparation **et/ou l'entretien de l'instrument médical ou dentaire** (50, 50A, 50B) grâce au premier dispositif de préparation **et/ou d'entretien (1);**
d) **la séparation de l'adaptateur de connexion (10), ensemble avec le** premier instrument médical ou dentaire (50, 50A, 50B) qui y est fixé, du premier dispositif de préparation **et/ou d'entretien (1);**
e) **le raccordement de l'adaptateur de connexion (10), ensemble avec** l'instrument médical ou dentaire (50, 50A, 50B) qui y est fixé, à un deuxième dispositif de préparation et/ou **d'entretien (2, 14) pour un instrument médical ou dentaire** (50, 50A, 50B), dans lequel les premier et deuxième dispositifs de préparation **et/ou d'entretien** (1; 2, 14) sont des dispositifs de préparation et/ou d'entretien différents qui appliquent différents traitements de préparation **et/ou d'entretien à l'instrument médical ou dentaire** (50, 50A, 50B);
f) la préparation **et/ou l'entretien de l'instrument médical ou dentaire** (50, 50A, 50B) grâce au deuxième dispositif de préparation **et/ou d'entretien (2, 14).**

2. **Procédé de préparation et/ou d'entretien selon la revendication 1, caractérisé en ce que l'étape b) comprend les étapes partielles suivantes:**
b1) **le raccordement de l'instrument médical ou dentaire** (50, 50A, 50B) à l'adaptateur de connexion (10);
**b2) le raccordement de l'adaptateur de connexion (10), ensemble avec l'instrument médical** ou dentaire (50, 50A, 50B) qui y est fixé, au premier dispositif de préparation et/ou **d'entretien (1).**

3. Procédé de préparation et/ou d'entretien selon la revendication 1 ou 2, **caractérisé en ce qu'après l'étape f), l'instrument médical ou dentaire** (50, 50A, 50B) **est séparé de l'adaptateur** de connexion (10).

4. **Procédé de préparation et/ou d'entretien selon l'une des revendications** précédentes, **caractérisé en ce que**
**l'instrument médical ou dentaire** (50, 50A, 50B) présente une enveloppe extérieure creuse (52) à l'intérieur de laquelle au moins un élément (53) est disposé, dans lequel la préparation **et/ou l'entretien de l'instrument médical** ou dentaire (50, 50A, 50B) grâce au premier dispositif de préparation et/ou **d'entretien (1) et/ou au** deuxième dispositif de préparation et/ou **d'entretien (2, 14) comprend une préparation et/ou un entretien de l'au moins un élément** (53) disposé **à l'intérieur de** l'enveloppe extérieure creuse (52), en particulier par conduction d'un **fluide de préparation et/ou ou d'entretien en direction de l'au moins un élément (53) ou vers** celui-ci.

5. **Procédé de préparation et/ou d'entretien selon l'une des revendications** précédentes, **caractérisé en ce que**
**l'adaptateur de connexion (10) et le premier dispositif de préparation et/ou d'entretien (1) et/ou l'adaptateur de connexion (10) et le deuxième dispositif de préparation et/ou d'entretien (2,** 14) sont connectés ensemble grâce à une connexion enfichable (3).

6. **Procédé de préparation et/ou d'entretien selon l'une des revendications précédentes, caractérisé en ce que**
**le premier dispositif de préparation et/ou d'entretien (1) comprend l'un des dispositifs suivants: un appareil** d'entretien pour la délivrance **d'un** fluide **d'entretien à l'instrument médical** ou dentaire (50, 50A, 50B); un appareil de préparation ou de nettoyage pour la délivrance **d'un** fluide **de préparation ou de nettoyage à l'instrument médical ou dentaire (50, 50A, 50B);** un appareil **de désinfection pour la délivrance d'un fluide de désinfection à l'instrument médical** ou dentaire (50, 50A, 50B); un stérilisateur ou autoclave pour la délivrance d'un fluide de stérilisation à l'instrument médical ou dentaire (50, 50A, 50B); et **en ce que** le deuxième dispositif de préparation et/ou d'entretien (2, 14) comprend l'un des dispositifs suivants, mais cependant pas un du genre identique au premier dispositif de préparation et/ou d'entretien (1): un appareil d'entretien pour la délivrance d'un fluide d'entretien à l'instrument médical ou dentaire (50, 50A, 50B); un appareil de préparation ou de nettoyage pour la délivrance d'un fluide de préparation ou de nettoyage à l'instrument médical ou dentaire (50, 50A, 50B); un appareil de désinfection pour la délivrance d'un fluide de désinfection à l'instrument médical ou dentaire (50, 50A, 50B); un stérilisateur ou autoclave pour la délivrance d'un fluide de stérilisation à l'instrument médical ou dentaire (50, 50A, 50B).

7. Procédé de préparation et/ou d'entretien selon l'une des revendications précédentes, **caractérisé en ce que**
les différents traitements de préparation et/ou d'entretien des premier et deuxième dispositifs de préparation et/ou d'entretien (1; 2, 14) comprennent l'alimentation de l'instrument médical ou dentaire (50, 50A, 50B) avec au moins un fluide de préparation et/ou d'entretien différent.

8. Système (100) de préparation et/ou d'entretien d'un instrument médical ou dentaire (50, 50A, 50B) dans un procédé selon l'une des revendications précédentes, avec: un premier dispositif de préparation et/ou d'entretien (1) pour un instrument médical ou dentaire, un deuxième dispositif de préparation et/ou d'entretien (2, 14) pour un instrument médical ou dentaire et un adaptateur de connexion (10) qui peut être raccordé de manière détachable à une extrémité de couplage (51) d'un instrument médical ou dentaire (50, 50A, 50B) et au premier dispositif de préparation et/ou d'entretien (1) pour un instrument médical ou dentaire et au deuxième dispositif de préparation et/ou d'entretien (2, 14) pour un instrument médical ou dentaire, **caractérisé en ce que**
le premier dispositif de préparation et/ou d'entretien (1) et le deuxième dispositif de préparation et/ou d'entretien (2, 14) sont des dispositifs de préparation et/ou d'entretien différents et appliquent différents processus de préparation et/ou d'entretien à l'instrument médical ou dentaire (50, 50A, 50B) de telle sorte que seul le premier dispositif de préparation et/ou d'entretien (1) ou le deuxième dispositif de préparation et/ou d'entretien (2, 14) est réalisé pour délivrer un lubrifiant à l'instrument médical ou dentaire (50, 50A, 50B) .

9. Système (100) de préparation et/ou d'entretien d'un instrument médical ou dentaire (50, 50A, 50B) selon la revendication 8, **caractérisé en ce que**
le premier dispositif de préparation et/ou d'entretien (1) et le deuxième dispositif de préparation et/ou d'entretien (2, 14) présentent respectivement au moins une pièce d'accouplement (4) à laquelle l'adaptateur de connexion (10) peut être raccordé grâce à une connexion enfichable (3).

10. Système (100) de préparation et/ou d'entretien d'un instrument médical ou dentaire (50, 50A, 50B) selon la revendication 8 ou 9, **caractérisé en ce que**
seulement l'un des dispositifs de préparation et/ou d'entretien (1, 2, 14) est réalisé pour délivrer un lubrifiant à l'instrument médical ou dentaire (50, 50A, 50B), et que l'autre dispositif de préparation et/ou d'entretien (1, 2, 14) est réalisé en tant que stérilisateur ou autoclave pour la délivrance d'un fluide de stérilisation à l'instrument médical ou dentaire (50, 50A, 50B).

11. Système (100) de préparation et/ou d'entretien d'un instrument médical ou dentaire (50, 50A, 50B) selon la revendication 8, 9 ou 10, **caractérisé par**
un dispositif de couplage (30) pour raccorder le premier et le deuxième dispositif de préparation et/ou d'entretien (1, 2, 14) pour la préparation et/ou l'entretien d'un instrument médical ou dentaire (50, 50A, 50B) à l'adaptateur de connexion (10) afin de relier par communication fluidique un instrument médical ou dentaire (50, 50A, 50B) à préparer et/ou entretenir aux premier et deuxième dispositifs de préparation et/ou d'entretien (1, 2, 14), dans lequel le dispositif de couplage (30) est réalisé en tant que connexion enfichable (3) et présente une pièce d'accouplement (4) prévue au niveau des premier et deuxième dispositifs de préparation et/ou d'entretien (1, 2, 14) ainsi qu'un élément d'accouplement (12) prévu au niveau de l'adaptateur de connexion (10), **caractérisé en ce que**
l'un des deux éléments, à savoir la pièce d'accouplement (4) ou l'élément d'accouplement (12), présente une saillie d'accouplement (13) et l'autre des deux éléments, à savoir la pièce d'accouplement (4) ou l'élément d'accouplement (12), un logement d'accouplement (5) pour le logement d'enfichage de la saillie d'accouplement (13), dans lequel celui des deux éléments, à savoir la pièce d'accouplement (4) ou l'élément d'accouplement (12), avec le logement d'accouplement (5) comprend: un corps (6) dans lequel un logement d'accouplement (5) est formé, au moins une conduite de fluide (7) pour la conduite d'un fluide de préparation et/ou d'entretien à travers le corps (6), dans lequel la conduite de fluide (7) se termine dans une ouverture (8) à une surface du logement d'accouplement (5), une plaque de verrouillage (9) disposée de manière mobile dans le corps (6), laquelle peut se mouvoir au choix dans une position de verrouillage et une position déverrouillée, dans lequel une ouverture de traversée (20) est ménagée dans la plaque de verrouillage (9), dans lequel l'ouverture de traversée (20) est en liaison fluidique avec l'ouverture (8) de la conduite de fluide (7) pour le passage du fluide de préparation et/ou d'entretien, un premier élément de précontrainte (21) pour précontraindre la plaque de verrouillage (9), une tige de verrouillage (22) qui est réceptionnée de manière déplaçable dans un alésage (23) du corps (6), dans lequel la tige de verrouillage s'étend à travers une ouverture de verrouillage (24) dans la plaque de verrouillage (9) et présente une pièce de verrouillage (25) et une pièce de déverrouillage (26), dans lequel l'ouverture de verrouillage (24) présente une partie de verrouillage (27) et une partie de déverrouillage (28), et un deuxième élément de précontrainte (29) pour précontraindre la tige de verrouillage (22) de telle sorte qu'une extrémité de contact (35) de la tige de verrouillage (22) fait saillie par rapport à la plaque de verrouillage (9), dans lequel la plaque de verrouillage (9) et la tige de verrouillage (22) coopèrent de telle sorte que, grâce à l'application d'une force à l'extrémité de contact (35) de la tige de verrouillage (22), l'extrémité de contact (35) est déplaçable à l'encontre de la précontrainte du deuxième élément de précontrainte (29) en direction de l'alésage (23) du corps (6) jusqu'à ce que la pièce de verrouillage (25) de la tige de verrouillage (22) est réceptionnée dans la partie de verrouillage (27) de l'ouverture de verrouillage (24), moyennant quoi, en raison de la force de précontrainte du premier élément de précontrainte (21), la plaque de verrouillage (9) est déplaçable dans la position de verrouillage, et dans lequel la plaque de verrouillage (9) et la tige de verrouillage (22) coopèrent de telle sorte que, grâce à l'application d'une force à la plaque de verrouillage (9) à l'encontre de la précontrainte du premier élément de précontrainte (21), la plaque de verrouillage (9) est déplacée dans la position déverrouillée, moyennant quoi, en raison de la force de précontrainte du deuxième élément de précontrainte (29), l'extrémité de contact (35) de la tige de verrouillage (22) peut être déplacée loin de l'alésage (23) du corps (6), moyennant quoi la pièce de déverrouillage (26) de la tige de verrouillage (22) est réceptionnée dans la partie de déverrouillage (26) de l'ouverture de verrouillage (24) et la plaque de verrouillage (9) est fixée dans la position déverrouillée.

12. Système (100) pour la préparation et/ou l'entretien d'un instrument médical ou dentaire (50, 50A, 50B) selon la revendication 11, **caractérisé en ce que**
le diamètre extérieur de la pièce de verrouillage (25) de la tige de verrouillage (22) est inférieur au diamètre extérieur de la pièce de déverrouillage (26) de la tige de verrouillage (22) et que le diamètre intérieur de la partie de verrouillage (27) de l'ouverture de verrouillage (24) est inférieur au diamètre intérieur de la partie de déverrouillage (28) de l'ouverture de verrouillage (24).

13. Système (100) de préparation et/ou d'entretien d'un instrument médical ou dentaire (50, 50A, 50B) selon l'une des revendications 11 ou 12, **caractérisé en ce**
**qu'**au niveau de l'autre élément, à savoir la pièce d'accouplement (4) ou l'élément d'accouplement (12), on prévoit avec la saillie d'accouplement (13) un retrait (36) dans lequel une arête périphérique (37) de l'ouverture de traversée (20) de la plaque de verrouillage (9) est en prise lorsque la saillie d'accouplement (13) est enfichée dans le logement d'accouplement (5) et la plaque de verrouillage (9) adopte la position de verrouillage, de sorte que la pièce d'accouplement (4) et l'élément d'accouplement (12) sont fixés l'un à l'autre.

14. Système (100) de préparation et/ou d'entretien d'un instrument médical ou dentaire (50, 50A, 50B) selon l'une des revendications 11 - 13, **caractérisé en ce**
**qu'**au niveau de l'adaptateur de connexion (10), un élément de raccordement (34) est prévu pour raccorder un instrument médical ou dentaire (50, 50A, 50B) à préparer et/ou à entretenir, dans lequel l'élément de raccordement (34) présente un écrou-raccord (33) avec un filetage intérieur (38) pour la liaison vissée avec un filetage (54) de l'instrument médical ou dentaire (50, 50A, 50B) , dans lequel l'écrou-raccord (33) est déplaçable le long d'un axe longitudinal (39) de l'élément de raccordement (34).

15. Système (100) de préparation et/ou d'entretien d'un instrument médical ou dentaire (50, 50A, 50B) selon l'une des revendications 11 - 14, **caractérisé en ce**
**qu'**à l'autre élément, à savoir la pièce d'accouplement (4) ou l'élément d'accouplement (12), on prévoit avec la saillie d'accouplement (13) une surface de contact (32) qui est agencée de telle sorte que, lors de l'enfichage de la saillie d'accouplement (13) dans le logement d'accouplement (5), elle produit l'application d'une force sur l'extrémité de contact (35) de la tige de verrouillage (22) pour déplacer l'extrémité de contact (35) à l'encontre de la précontrainte du deuxième élément de précontrainte (29) en direction de l'alésage (23) du corps (6) jusqu'à ce que la pièce de verrouillage (25) de la tige de verrouillage (9) est réceptionnée dans la partie de verrouillage (27) de l'ouverture de verrouillage (24), moyennant quoi en raison de la précontrainte du premier élément de précontrainte (21), la plaque de verrouillage (9) est déplacée dans la position de verrouillage.
